# EUROPEAN PATENT SPECIFICATION

(11) **EP 3 777 797 B1**
(45) Date of publication and mention of the grant of the patent: **31.01.2024**
(21) Application number: 19781337.1
(22) Date of filing: 11.01.2019
(51) Int. Cl.: A61F 13/15, A61F 13/49, A61F 13/494

(54) **METHOD FOR PRODUCING HYGIENE PRODUCT, AND HYGIENE PRODUCT**
VERFAHREN ZUR HERSTELLUNG EINES HYGIENEPRODUKTS UND HYGIENEPRODUKT
PROCÉDÉ DE PRODUCTION D'UN PRODUIT D'HYGIÈNE, ET PRODUIT D'HYGIÈNE

(30) Priority: 06.04.2018 JP 2018073688
(43) Date of publication of application: 17.02.2021
(73) Proprietor: Unicharm Corporation, Shikokuchuo-shi, Ehime 799-0111 (JP)
(72) Inventor: ICHIKAWA, Makoto, Kanonji-shi, Kagawa 769-1602 (JP); KAWABATA, Kuniyoshi, Kanonji-shi, Kagawa 769-1602 (JP)
(74) Representative: Staeger & Sperling Partnerschaftsgesellschaft mbB
(86) International application number: PCT/JP2019/000780
(87) International publication number: WO 2019/193804

(56) References cited:
- EP-A1- 2 266 513
- EP-A1- 2 478 880
- WO-A1-2009/123213
- JP-A- 2006 175 007
- JP-A- 2007 312 963
- JP-A- 2008 284 059
- JP-A- 2009 207 660
- JP-A- 2009 207 660
- JP-A- 2009 261 906

## Description

### FIELD

The present invention relates to a manufacturing method of a hygiene product, and a hygiene product.

### BACKGROUND

A hygiene product which includes a sheet-like functional member, wherein the functional member includes on one surface in a thickness direction, a pair of folded portions which extend along a longitudinal direction, are arranged side by side in a width direction, and are folded in the thickness direction, wherein a region between both end portions in the longitudinal direction in each of the pair of folded portions is movable, is known. As such a hygiene product, for example, an absorbent article such as a disposable diaper and a sanitary napkin, or a medical article such as a mask, may be mentioned. For example, the absorbent article described in Patent Literature 1 (Japanese Unexamined Patent Publication No. 2011 - 10839 : EP2450015(A1)) includes an absorbent main body portion in which a top sheet, an absorbent body, and a back sheet are laminated in this order, as a sheet-like functional member. The absorbent main body portion includes a pair of leakage prevention walls as a pair of folded portions. In each of the pair of leakage prevention walls, the region between both end portions in the longitudinal direction are set to be movable, and the pair of leakage prevention walls stand up in a wall-like manner so as to suppress the leakage of excretion. Further, the mask described in for example Patent Literature 2 (Japanese Unexamined Patent Publication No. 2012 - 217651) includes a mask main body portion which has a single or a plurality of pieces of sheet-like nonwoven fabric including a filter sheet, as a sheet-like functional member. The mask main body portion includes a pair of folds as a pair of folded portions. In each of the pair of folds, the region between both end portions in the longitudinal direction are set to be movable, and the folds are deformed into a bellows shape so as to form a predetermined mouth space between the inner surface of the mask main body portion and the wearer.

### [CITATION LIST]

### [PATENT LITERATURE]

[PTL 1] Japanese Unexamined Patent Publication No. 2011 - 10839
[PTL 2] Japanese Unexamined Patent Publication No. 2012 - 217651
Further prior art in this technical filed is disclosed in documents WO 2009/123213 A1, EP 2 266 513 A1, JP 2009/207660 A and EP 2 478 880 A1.

### SUMMARY

### [TECHNICAL PROBLEM]

In the manufacturing method of the above-mentioned hygiene product, at least a folded portion forming process, a cutting process, a rotating process, and a conveying process are sequentially performed. The folded portion forming process conveys a continuous sheet member in which the plurality of functional members are connected in the machine direction so that the longitudinal direction, the width direction, and the thickness direction of the functional member correspond to the machine direction, a cross machine direction, and a transverse direction, respectively of a manufacturing apparatus. Further, this process forms on one surface in the transverse direction of the continuous sheet member, the pair of folded portions which extend along the machine direction, are arranged side by side in the cross machine direction, and are folded in the thickness direction, for each of the functional members. Subsequently, the cutting process cuts the continuous sheet member along the cross machine direction for each of the functional members. Subsequently, the rotating process rotates each of the cut functional members so that the width direction corresponds to the machine direction, that is, rotates the same by 90°. Subsequently, the conveying process conveys the functional members in the machine direction toward the processes in the later stage (on the downstream), in a state in which a surface on an opposite side of the surface on which the pair of folded portions are formed in the functional members is set as a conveying surface, that is, in a state in which the pair of folded portions are exposed.

Accordingly, after the rotating process, since the longitudinal direction, the width direction, and the thickness direction of the functional member correspond to the cross machine direction, the machine direction, and the transverse direction, respectively of the manufacturing apparatus, the pair of folded portions are provided in a state of extending along the cross machine direction. In other words, the pair of folded portions are conveyed in a state of being almost orthogonal to the machine direction. At this time, since the conveyed functional members move in the conveying speed with respect to the surrounding air, the wind with the conveying speed is relatively blown against the functional members. Accordingly, since the pair of folded portions are in a state of relatively easily receiving the wind pressure during the conveyance, there may be cases in which the region between the both end portions in the longitudinal direction in the folded portions which is formed so as to be movable moves and is deformed by the wind pressure, and for example may be turned over in an unexpected direction. Then, it is possible that the folded portions which are turned over in the unexpected direction may deviate from the planned position or shape due to the folding and joining in the processes in the later stage (on the downstream), for example, in the process of folding the functional members, and the process of joining the members which include the functional members with each other, etc. When the folded portions deviate from the planned position or shape, depending on the degree of deviation, there may be cases in which products cannot be shipped due to defective products, or even if they were not defective products, the appearance of the products when being used may be deteriorated.

Further, even when deformation such as turning over does not occur in the folded portions when being manufactured, there may be cases in which deformation such as turning over occur in the folded portions by receiving vibration or force from outside during the transport or storage of hygiene products. Then, the folded portions cannot demonstrate the predetermined function properly from the beginning of usage, and cannot maintain the appearance as a product with high quality.

Accordingly, the object of the present invention is to provide a manufacturing method of a hygiene product which includes a sheet-like functional member that includes a pair of folded portions, and which is capable of suppressing the deformation such as turning over, etc., of the folded portions while being manufactured. Further, another object of the present invention is to provide a hygiene product which includes a sheet-like functional member that includes a pair of folded portions, and which is capable of suppressing the deformation of the folded portions while the hygiene product is being stored.

### [SOLUTION TO PROBLEM]

The manufacturing method of the hygiene product of the present invention is defined in claim 1 and is a manufacturing method of a hygiene product which includes a sheet-like functional member, wherein the functional member includes on one surface in a thickness direction, a pair of folded portions which extend along a longitudinal direction, are arranged side by side in a width direction, and are folded in the thickness direction, and a region between both end portions in the longitudinal direction in each of the pair of folded portions is movable, the manufacturing method including: a folded portion forming process, while conveying in a machine direction, a continuous sheet member in which the plurality of functional members are connected in the machine direction so that the longitudinal direction, the width direction, and the thickness direction correspond to the machine direction, a cross machine direction, and a transverse direction, respectively of a manufacturing apparatus, of forming on one surface in the transverse direction of the continuous sheet member, the pair of folded portions which extend along the machine direction, are arranged side by side in the cross machine direction, and are folded in the transverse direction, for each of the functional members, a temporarily fixing process of forming a temporarily fixed portion in a region between both end portions in the machine direction in at least one of the pair of folded portions, for each of the functional members of the continuous sheet member, a cutting process of cutting the continuous sheet member along the cross machine direction for each of the temporarily fixed functional members, a rotating process of rotating each of the cut functional members so that the width direction corresponds to the machine direction, and a conveying process of conveying each of the rotated functional members in the machine direction, in a state in which a surface on an opposite side of a surface on which the pair of folded portions are formed in each of the rotated functional members is set as a conveying surface.

In the present manufacturing method of a hygiene product, before rotating the functional member so that the width direction corresponds to the machine direction, that is, rotating the same by 90°, the region between the both end portions in the longitudinal direction (the machine direction) in at least one of the pair of folded portions is temporarily fixed. Accordingly, since the region between the both end portions in the longitudinal direction which can easily move in at least one of the pair of folded portions is temporarily fixed, after the at least one of the pair of folded portions are rotated by 90°, even when the same is brought to a state in which it is relatively easy to receive wind pressure during conveyance, it becomes difficult to move, for example, to be turned over. Accordingly, at least one of the pair of folded portions can be suppressed from, for example, being turned over in an unexpected direction by the wind pressure. That is, each of the folded portions can be suppressed from being deviated from the planned position or shape due to the folding and joining in the processes in the later stage, for example, in the process of folding the functional member, and the process of joining the members which include the functional member with each other, etc. Therefore, cases in which products cannot be shipped due to defective products, or cases in which the appearance of the products when being used may be deteriorated can be suppressed. Incidentally, since the region between the both end portions in the longitudinal direction in at least one of the pair of folded portions is only temporarily fixed, the wearer can easily release the temporal fixing when using the hygiene product.

The manufacturing method of the hygiene product of the present invention further comprises the features that the temporarily fixing process includes a process of forming, in the machine direction, the temporarily fixed portion in a region between fixed portions which fix at least one of the pair of the folded portions to each of the functional members, positioned on the both end portions in the at least one of the pair of folded portions.

In the present manufacturing method of a hygiene product, in the machine direction, that is, in the longitudinal direction of the functional member, a region between fixed portions which are positioned on the both end portions in the pair of folded portions is temporarily fixed. Accordingly, it becomes difficult in a greater degree for the movable portion in the folded portions to move across the entire longitudinal direction of the functional member, whereby it becomes more difficult for the same to be turned over.

In an advantageous embodiment of the manufacturing method of the hygiene product of the present invention the rotating process includes a process of rotating each of the functional members, while retaining each of the functional members using an adsorption means by making a surface on which the pair of folded portions are positioned in each of the functional members be adsorbed by the adsorption means of a rotation apparatus, by rotating the adsorption means.

In the present manufacturing method of a hygiene product, in the rotating process, it is easy for the folded portions to move and to be turned over by the centrifugal force and the wind pressure at the time of rotation. That is, even when the entire functional member is moving in the predetermined speed and the turning over has not occurred in the folded portions, in a case in which the machine direction is rotated by 90°, since the folded portions are displaced in the direction of the wind while increasing the speed in accordance with the rotation, it is easier for the turning over to occur compared to the state of being conveyed before and after the rotation. Accordingly, in the present manufacturing method, after the folded portions are temporarily fixed, the surface on which the pair of folded portions are positioned in the functional member is adsorbed by the adsorption means, so as to be rotated while retaining the functional member. Accordingly, in the present manufacturing method, it becomes difficult for the folded portions to move and the turning over to occur by the temporal fixing and the adsorption in the folded portions, even during the rotating process.

Further, when the surface on which the folded portions are positioned in the functional member is separated from the adsorption means, there may be cases in which the folded portions are pulled by the adsorption means, so that the folded portions move and the turning over occurs. However, in the present manufacturing method, since the folded portions are temporarily fixed, even when the folded portions are pulled by the adsorption means, the folded portions can be suppressed from being moved and the turning over can be suppressed from occurring.

In an additional advantageous embodiment of the manufacturing method of the hygiene product of the present invention, the manufacturing method of the hygiene product according, further comprises a folding process, after the conveying process, while making a surface on which the pair of folded portions are positioned in each of the functional members in contact with a folding means of a folding apparatus, of folding the surface along a folding line along the machine direction.

In the present manufacturing method of a hygiene product, the functional member is folded while the folding means of the folding apparatus is in contact with the pair of folded portions, whereby it is easy for the folded portions to move and the turning over to occur. However, in the present manufacturing method, since the folded portions are temporarily fixed, it becomes difficult for the folded portions to move and for the turning over to occur even in such cases.

In an additional advantageous embodiment of the manufacturing method of the hygiene product of the present invention in a region between the both end portions in the longitudinal direction of at least one of the pair of folded portions, one end portion in the cross machine direction is a fixed end, and the other end portion is a movable end, and the temporarily fixing process includes a process of forming the temporarily fixed portion in a region closer to the movable end than the fixed end.

In the present manufacturing method of a hygiene product, the temporarily fixed portion is formed in a region close to the movable end in the folded portions. Since it is easier for the region close to the movable end in the folded portions to move and to turn over, compared to the region close to the fixed end, the temporarily fixed portion is formed in such a region, whereby it becomes difficult for the movable portion of the folded portions to move, and for the turning over to occur.

According to the invention the hygiene product is an absorbent article, the functional member is an absorbent main body portion which includes a top sheet, a back sheet, and an absorbent body which is positioned between the top sheet and the back sheet, and the pair of folded portions are a pair of leakage prevention walls.

In the present manufacturing method of a hygiene product, the pair of leakage prevention walls in the absorbent main body portion may be brought to a state in which it is relatively easy to receive wind pressure during conveyance after the rotating process. However, in the present manufacturing method, since the leakage prevention walls are temporarily fixed, the folding and the joining can be suppressed from being deviated from the planned position or shape in the later stage, for example, in the process of folding the absorbent main body portion, and the process of joining the members which include the absorbent main body portion with each other, etc. Therefore, cases in which products cannot be shipped due to absorbent articles being defective products, or cases in which the appearance of the products when being used may be deteriorated can be suppressed. Further, since the leakage prevention walls are only temporarily fixed, the temporal fixing can be easily released and can be moved at the time of usage.

The hygiene product of the present invention is defined in claim 5 and is a hygiene product which includes a sheet-like functional member, wherein the functional member includes on one surface in a thickness direction, a pair of folded portions which extend along a longitudinal direction, are arranged side by side in a width direction, and are folded in the thickness direction, and a region between both end portions in the longitudinal direction in each of the pair of folded portions is movable, including: a temporarily fixed portion which temporarily fixes a region between the both end portions in the longitudinal direction in at least one of the pair of folded portions.

In the present hygiene product, at least one of the folded portions is temporarily fixed after the hygiene product is manufactured until the beginning of usage, that is, at least one of the folded portions is temporarily fixed to another portion. Accordingly, at least the temporarily fixed folded portion is not deformed during the storage of the hygiene product, and can demonstrate the predetermined function properly from the beginning of usage. Further, at least one of the folded portions is temporarily fixed, whereby at least the temporarily fixed folded portion is not deformed during the storage of the hygiene product, and thus the straight line portion of the fold is properly retained, and can maintain the appearance as a product with high quality.

### [ADVANTAGEOUS EFFECTS OF INVENTION]

According to the present invention, in the manufacturing method of a hygiene product which includes a sheet-like functional member that includes a pair of folded portions, the deformation such as turning over, etc., of the folded portions while being manufactured can be suppressed. Further, according to the present invention, in a hygiene product which includes a sheet-like functional member that includes a pair of folded portions, the deformation of the folded portions while the hygiene product is being stored can be suppressed.

### BRIEF DESCRIPTION OF DRAWINGS

[FIG. 1] FIG. 1 is a plan view which shows a configurational example of the disposable diaper according to an embodiment.
[FIG. 2] FIG. 2 is a cross sectional view along the II - II line of FIG. 1.
[FIG. 3] FIG. 3 is a cross sectional view along the II - II line of FIG. 1, which shows a modification example of the configuration of the disposable diaper according to the embodiment.
[FIG. 4] FIG. 4 is a plan view which shows a modification example of the configuration of the absorbent main body portion of the disposable diaper according to the embodiment.
[FIG. 5] FIG. 5 is a schematic view which shows the manufacturing method of the disposable diaper according to the embodiment.
[FIG. 6] FIG. 6 is another schematic view which shows the manufacturing method of the disposable diaper according to the embodiment.
[FIG. 7] FIG. 7 is still another schematic view which shows the manufacturing method of the disposable diaper according to the embodiment.
[FIG. 8] FIG. 8 is a schematic view which shows the adsorption state of the absorbent main body portion in the rotating process of the manufacturing method of the disposable diaper according to the embodiment.
[FIG. 9] FIG. 9 is a plan view and a cross sectional view which show the configurational example of a mask according to another embodiment.

### DESCRIPTION OF EMBODIMENTS

Hereinbelow, the manufacturing method of a hygiene product and the hygiene product according to an embodiment of the present invention will be explained by mentioning a pants-type disposable diaper (hereinbelow, which may also be referred to simply as "a disposable diaper ") which is one type of an absorbent article as an example of the hygiene product. Note that the present invention is not limited to such an example, and the present invention may be applied to various hygiene products without departing from the scope of the subject matter of the present invention. As the hygiene products, other absorbent articles and medical articles may be mentioned. As other absorbent articles, for example, other types of disposable diapers, incontinence pads, and sanitary napkins may be mentioned, and as the medical articles, for example, masks may be mentioned.

First, the disposable diaper 1 according to the present embodiment is explained.

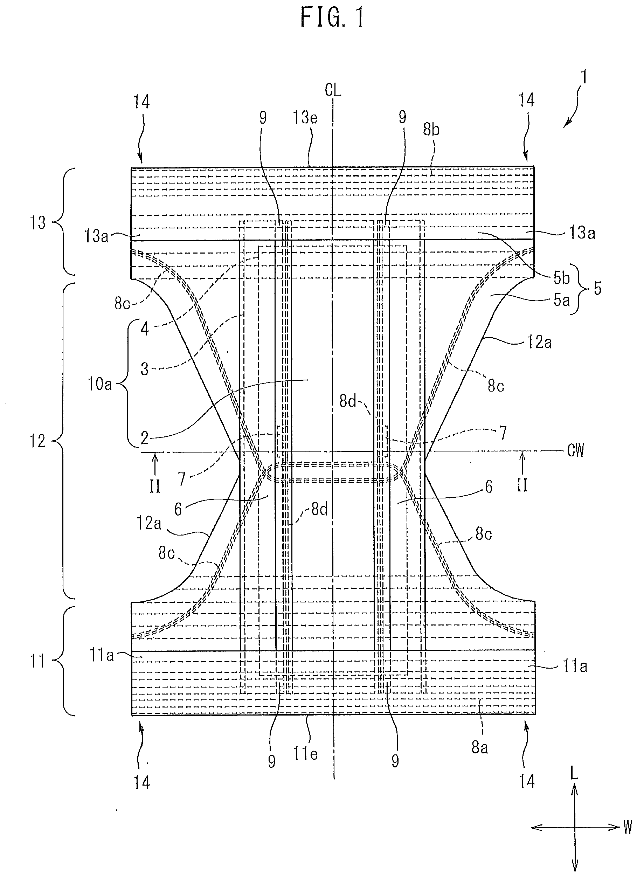

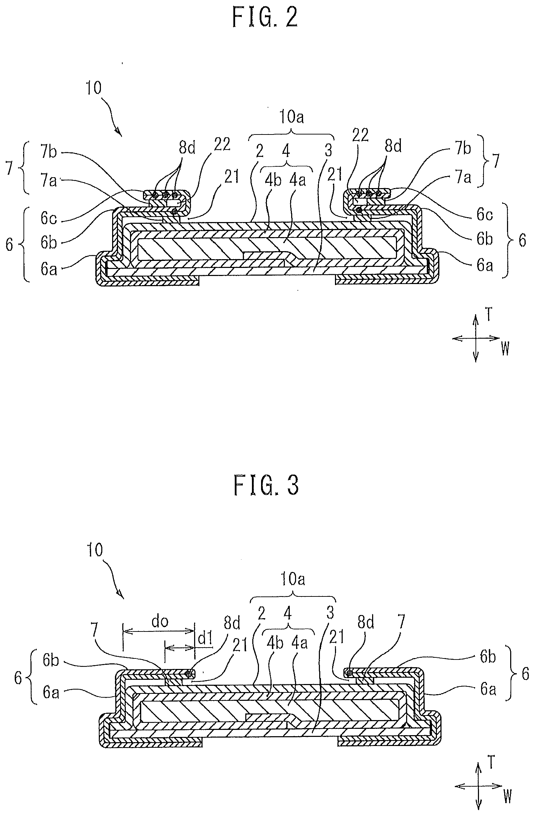

FIG. 1 and FIG. 2 are views which show the configurational example of the disposable diaper 1 according to the present embodiment. Note that FIG. 1 is a plan view which shows the expanded state of the disposable diaper 1, and FIG. 2 is a cross sectional view along the II - II line of FIG. 1. The disposable diaper 1 includes, in the state as shown in FIG. 1 and FIG. 2, the longitudinal direction L, the width direction W, and the thickness direction T, which are mutually orthogonal to each other. Further, the disposable diaper 1 includes the longitudinal direction center line CL which passes the center of the diaper in the width direction W and extends in the longitudinal direction L, and the width direction center line CW which passes the center of the diaper in the longitudinal direction L and extends in the width direction W. Still further, the direction which moves closer to and the direction which moves farther from the longitudinal direction center line CL are regarded as the direction on the inner side and the direction on the outer side in the width direction W, respectively. The direction which moves closer to and the direction which moves farther from the width direction center line CW are regarded as the direction on the inner side and the direction on the outer side in the longitudinal direction L, respectively. Viewing the disposable diaper 1 which is placed on a plane that includes the longitudinal direction L and the width direction W from the upper side in the thickness direction T is referred to as "a plan view", and the shape which is grasped in a plan view is referred to as "a planar shape". "The skin side" and "the non - skin side" mean, at the time of wearing the disposable diaper 1, the side relatively closer to the skin surface of the wearer and the side farther from the skin surface, respectively, in the thickness direction T of the disposable diaper 1. The definitions of these directions, etc., are applied not only to the disposable diaper 1 in the state of FIG. 1, but also to the disposable diaper 1 in a state folded at the folding line along the width direction center line CW, and to the materials which configure the disposable diaper 1.

The disposable diaper 1 is a pants type diaper which includes the ventral side portion 11, the dorsal side portion 13, and the crotch portion 12 arranged between the ventral side portion 11 and the dorsal side portion 13. The ventral side portion 11 is a portion in the disposable diaper 1 which is applied to the ventral portion of the wearer. The crotch portion 12 is a portion in the disposable diaper 1 which is applied to the crotch portion of the wearer. The dorsal side portion 13 is a portion in the disposable diaper 1 which is applied to the hip portion and / or the dorsal portion of the wearer. The both end portions 11a, 11a in the width direction W of the ventral side portion 11 and the both end portions 13a, 13a in the width direction W of the dorsal side portion 13 are joined to each other, respectively, by the pair of joining portions 14, 14 in a state of being overlapped in the thickness direction T along the longitudinal direction L. In the disposable diaper 1, the waist opening portion WO is formed by the end portion 11e on the opposite side of the crotch portion 12 in the longitudinal direction L in the ventral side portion 11, and the end portion 13e on the opposite side of the crotch portion 12 in the longitudinal direction L in the dorsal side portion 13. In the disposable diaper 1, the pair of leg opening portions LO, LO are formed by the both side portions 12a, 12a in the width direction W in the crotch portion 12.

The disposable diaper 1 includes the absorbent main body portion 10. The absorbent main body portion 10 has, in the present embodiment, in a plan view, a substantially rectangular shape which includes the longer side that extends in the longitudinal direction L and the shorter side that extends in the width direction W. Note that the shape of the absorbent main body portion 10 is not limited to this example, and the absorbent main body portion 10 may have any long shape as long as it is a shape in which the length dimension in the longitudinal direction L is longer than the width dimension in the width direction W. As the long shape, for example, a rectangular shape in which both edges in the longitudinal direction L are bulged in an arc-shape, an ellipse shape, a gourd shape, and an hourglass shape, may be mentioned.

The absorbent main body portion 10 includes the top sheet 2, the back sheet 3, the absorbent body 4 which is positioned between the top sheet 2 and the back sheet 3, and the pair of side sheets 6, 6.

The top sheet 2 has the same planar shape as the absorbent main body portion 10 does, and is positioned on the skin side of the absorbent main body portion 10. The top sheet 2 is a sheet which has a liquid permeability. The top sheet 2 is not particularly limited as long as it is a sheet which has a liquid permeability. As the top sheet 2, for example, a liquid permeable nonwoven fabric, a liquid permeable woven fabric, a synthetic resin film with liquid permeable holes, and a composite sheet of the aforementioned, may be mentioned. In the present embodiment, an air through nonwoven fabric which has a liquid permeability is used. As the basis weight of the top sheet 2, for example, 5 g / m² to 100 g / m² may be mentioned. As the thickness of the top sheet 2, for example, 0.2 mm to 3 mm may be mentioned.

The back sheet 3 has the same planar shape as the absorbent main body portion 10 does, and is positioned on the non - skin side of the absorbent main body portion 10. The back sheet 3 is a sheet which has a liquid impermeability. The back sheet 3 is not particularly limited as long as it is a sheet which has a liquid impermeability, and for example, a liquid impermeable nonwoven fabric, a synthetic resin film, a composite sheet of a nonwoven fabric and a synthetic resin film, an SMS nonwoven fabric may be mentioned. In the present embodiment, an SMS nonwoven fabric which has a liquid impermeability is used.

The absorbent body 4 has a similar planar shape as the absorbent main body portion 10 does, and is enclosed between the top sheet 2 and the back sheet 3. The surface on the non - skin side of the top sheet 2 and the surface on the skin side of the absorbent body 4 are joined to each other, and the surface on the non - skin side of the absorbent body 4 and the surface on the skin side of the back sheet 3 are joined to each other. The peripheral portion of the surface on the non - skin side of the top sheet 2 and the peripheral portion of the surface on the skin side of the back sheet 3 are joined to each other. The joining is formed by using for example an adhesive agent, and as the adhesive agent, a known material, for example, a hot melt adhesive agent, may be used.

The absorbent body 4 is a layer which has a liquid absorbency and liquid retention property, and includes, for example, superabsorbent polymers and hydrophilic fibers. As the thickness of the absorbent body 4, for example; 1 mm to 20 mm may be mentioned. In the present embodiment, the absorbent body 4 includes the absorbent material 4a and the liquid permeable sheet 4b that incorporates the absorbent material 4a. The absorbent material 4a includes superabsorbent polymers and hydrophilic fibers, especially, absorbent fibers. The superabsorbent polymers are not particularly limited as long as they can absorb and retain moisture, and for example, acrylic acid based superabsorbent polymers (SAP) may be mentioned. As the basis weight of the superabsorbent polymers, for example, 10 to 500 g / m² may be mentioned. The absorbent fibers are not particularly limited as long as they are hydrophilic fibers which can absorb and retain moisture, and for example, pulp fibers may be mentioned. As the basis weight of the absorbent fibers, for example, 10 to 500 g / m² may be mentioned. The liquid permeable sheet 4b is not particularly limited as long as it is a sheet which has a liquid permeability, and a liquid permeable nonwoven fabric, laminated nonwoven fabric, and a hydrophilic sheet such as tissue may be mentioned. The liquid permeable sheet 4b may have a configuration in which a plurality of the aforementioned sheets are laminated. As the basis weight of the liquid permeable sheet 4b, for example, 2 g / m² to 80 g / m² may be mentioned.

The pair of side sheets 6, 6 are arranged so as to cover the surface on both sides in the width direction W in the top sheet 2, and are liquid impermeable sheets which extend in the longitudinal direction L. Both of the pair of end portions which face each other toward the inner side in the width direction W in the pair of side sheets 6, 6 are folded in the thickness direction T so as to be capable of standing up on the surface on both sides in the width direction W in the top sheet 2, and the edge on the inner side in the width direction W is set to be a free end (the movable end) so as to form a leakage prevention wall.

In the present embodiment, the portion on the inner side in the width direction W among each of the pair of side sheets 6, 6 is the leakage prevention wall which extends in the longitudinal direction L, and the portion on the outer side in the width direction W is the protection member of the lamination body 10a which is arranged so as to cover the end portion on the outer side in the width direction W in the lamination body 10a of the top sheet 2, the absorbent body 4, and the back sheet 3, and is the fixed member of the leakage prevention wall. At the both end portions in the longitudinal direction L in each of the pair of side sheets 6, 6, the fixed portions 9, 9 which fix the both end portions in the longitudinal direction L of the leakage prevention wall to the absorbent main body portion 10.

To be specific, each the pair of side sheets 6, 6 includes the covered portion 6a, and the leakage prevention portions 6b, 6c which extend along the longitudinal direction L and are adjacent to each other in the width direction W. The covered portion 6a on the outer side in the width direction W in the side sheet 6 is joined to the lamination body 10a at the end portion on the outer side in the width direction W of the lamination body 10a so as to cover the edge in the width direction W of the back sheet 3 from the surface on the non - skin side of the back sheet 3, and further to cover the surface on the skin side of the top sheet 2 from the edge in the width direction W of the top sheet 2. The leakage prevention portion 6b is adjacent on the inner side in the width direction W of the covered portion 6a, and is folded in a contacting or non-contacting state on the upper side in the thickness direction T of the top sheet 2. In the leakage prevention portion 6b, the end portion on the outer side in the width direction W is fixed to the covered portion 6a (the end portion being the fixed end), and the end portion on the inner side is not fixed to the top sheet 2, etc., so as to be in a state of being able to easily move by an external force, that is, the end portion is movable (the end portion being a free end or a movable end). The leakage prevention portion 6c is adjacent on the inner side in the width direction W of the leakage prevention portion 6b, and is folded in a contacting or non-contacting state on the upper side in the thickness direction T of the leakage prevention portion 6b. In the leakage prevention portion 6c, the end portion on the inner side in the width direction W is connected to the leakage prevention portion 6b and is movable together with the leakage prevention portion 6b, however, is fixed with respect to the leakage prevention portion 6b (the end portion being the fixed end), and the end portion on the outer side is not fixed to other portions or the top sheet 2, etc., so as to be movable (the end portion being a free end or a movable end). At both end portions in the longitudinal direction L in each of the pair of side sheets 6, 6, the fixed portions 9, 9 which fix the both end portions in the longitudinal direction L in the leakage prevention portions 6b, 6c to the absorbent main body portion 10 are formed.

Accordingly, it can be said that the pair of leakage prevention portions 6b, 6b and / or the pair of leakage prevention portions 6c, 6c in the pair of side sheets 6, 6 are the pair of folded portions which extend along the longitudinal direction L, are arranged side by side in the width direction W, and are folded in the thickness direction T, on one surface in the thickness direction T of the absorbent main body portion 10.

It should be noted that the configuration of the pair of side sheets 6, 6 is not limited to the configuration as shown in FIG. 2, and another configuration may be applied as long as the leakage prevention portions extend in the longitudinal direction L, one end portion in the width direction W of the leakage prevention portions is fixed (for example : the fixed end), and the other end portion is in a state of being able to move (for example : the free end or the movable end), whereby the leakage prevention walls can be configured. FIG. 3 is a cross sectional view along the II - II line of FIG. 1, which shows a modification example of the configuration of the disposable diaper 1. The configuration of this modification example differs from the configuration as shown in FIG. 2 in that although each of the pair of side sheets 6, 6 includes the covered portion 6a, and the leakage prevention portion 6b, the same does not include the leakage prevention portion 6c. That is, in the case of FIG. 2, a portion of the movable portion in the side sheet 6 is folded back so as to configure two types of the leakage prevention portions 6b, 6c, whereas in the case of FIG. 3, the movable portion in the side sheet 6 configures one type of the leakage prevention portion 6b. Alternatively, a configuration may be applied in which one or a plurality of leakage prevention walls which are folded on the leakage prevention portion 6c are further connected to the end portion on the outer side in the width direction W of the leakage prevention portion 6c in the configuration of FIG. 2.

The absorbent main body portion 10 further includes the temporarily fixed portion 7 which temporarily fixes the region between the both end portions in the longitudinal direction L in at least one of the pair of leakage prevention portions (the pair of folded portions) in the pair of side sheets. The temporarily fixed portion 7 temporarily fixes the movable portion in the leakage prevention wall, so as to suppress the movement thereof. The temporarily fixed portion 7 may take any position, any number, and any shape in a plan view, as long as it is formed in the region between the both end portions in the longitudinal direction L in the leakage prevention portions and can suppress the movement of the leakage prevention portions.

In a plan view, the number of the temporarily fixed portions 7 which are formed in the region between the both end portions in the longitudinal direction L in the leakage prevention portion is, from the viewpoint of reliably performing the temporal fixing and of being able to easily remove the temporarily fixed portions 7 at the time of usage, preferably n or less (wherein n ≤ 5), and more preferably one. For example, when n number of temporarily fixed portions 7 are provided in the longitudinal direction L in a plan view, the temporarily fixed portions 7 are arranged at equally divided points when the leakage prevention portion is substantially equally divided into (n+1) in the longitudinal direction L.

In a case in which the temporarily fixed portion 7 is provided at one position in the leakage prevention portion, from the viewpoint of reliably performing the temporal fixing, as the position in the longitudinal direction L and the width direction W of the temporarily fixed portion 7 (the position of the center of gravity), the following position may be mentioned. That is, first, as the position in the longitudinal direction L, when the length in the longitudinal direction L of the absorbent main body portion 10 is regarded as 100 %, the above-mentioned position is preferably arranged at a range within ± 30% in the longitudinal direction L from the width direction center line CW, and is more preferably arranged at a range within ± 10% therein. Further, as the position in the width direction W, when the width in the width direction W of the leakage prevention portion is regarded as 100 %, the above-mentioned position is preferably arranged at a range + 5 % or more and within 60 % in the width direction W from the edge on the movable end side, and is more preferably arranged at a range + 10 % or more and within 40 % therein.

As the individual shape in a plan view of the temporarily fixed portions 7, for example, circle, oval (rounded rectangle), ellipse, rectangle, polygon, star, linear, etc., may be mentioned. Further, the portion of a plurality of temporarily fixed portions each having such a shape may be arranged altogether in a specific region and may be regarded as one temporarily fixed portion 7. In such a case, as the arrangement pattern of the plurality of portions, a pattern of arranging the same along the outer shape of, for example, circle, oval (rounded rectangle), ellipse, rectangle, polygon, star, linear, etc., may be mentioned.

As the temporarily fixed portion 7, for example, an adhesive agent with a relatively weak joining strength may be mentioned. In the present embodiment, as the temporarily fixed portion 7, a non-tacky hot melt adhesive agent is used. It should be noted that the temporarily fixed portion 7 is not limited to this example, and the material sheets may be melted and joined with each other by methods such as heat sealing, ultrasonic sealing, and mechanical crimping (pressing), etc. In such cases, the melted and joined portion is to be the temporarily fixed portion 7. Incidentally, in a case in which methods such as heat sealing, ultrasonic sealing, and mechanical crimping (pressing) are used, it is preferable that the temporarily fixed portion 7 is formed at the region which does not overlap in a plan view with the elastic member 8d (which will be described later) arranged in the side sheet 6. This is from the viewpoint of not suppressing the function of the elastic member 8d.

In the present embodiment, the absorbent main body portion 10 includes, in each of the side sheets 6, at the center portion in the longitudinal direction L in the region between the both end portions in the longitudinal direction L (between the fixed portions 9, 9), that is, at the range within ± 5 % in the longitudinal direction L from the width direction center line CW, the temporarily fixed portion 7 which is formed by a non-tacky hot melt adhesive agent. The temporarily fixed portion 7 includes the temporarily fixed portion 7a and the temporarily fixed portion 7b. The temporarily fixed portion 7a temporarily fixes the movable leakage prevention portion 6b to the top sheet 2. The temporarily fixed portion 7b temporarily fixes the movable leakage prevention portion 6c to the leakage prevention portion 6b, whereby substantially temporarily fixes the same to the top sheet 2. The temporarily fixed portion 7a and the temporarily fixed portion 7b are overlapped with each other at least a portion thereof in a plan view. The both temporarily fixed portions are arranged close to each other in a plan view in this manner, whereby when the disposable diaper 1 is used, the both temporarily fixed portions can be removed almost at the same time. It should be noted that the both temporarily fixed portions may be apart from each other in a plan view.

It should be noted that in the present embodiment, the leakage prevention portion has only to include the temporarily fixed portion 7 in at least one side sheet among the pair of side sheets 6, 6. Further, the temporarily fixed portion 7 has only to be included in at least one leakage prevention portion among the leakage prevention portion 6b and the leakage prevention portion 6c, in each of the side sheets 6. That is, the leakage prevention portion 6b and / or 6c in the side sheet 6 on one side includes the temporarily fixed portion 7a and / or 7b which temporarily fixes the region between the both end portions in the longitudinal direction L. And / alternatively, the leakage prevention portion 6b and / or 6c in the side sheet 6 on the other side includes the temporarily fixed portion 7a and / or 7b which temporarily fixes the region between the both end portions in the longitudinal direction L.

In the modification example of the configuration of the disposable diaper 1 as shown in FIG. 3, the temporarily fixed portion 7 is formed, in each of the side sheets 6, at the center portion in the longitudinal direction L in the region between the both end portions in the longitudinal direction L, that is, at the range within ± 5 % in the longitudinal direction L from the width direction center line CW, by a non-tacky hot melt adhesive agent, and temporarily fixes the movable leakage prevention portion 6b to the top sheet 2.

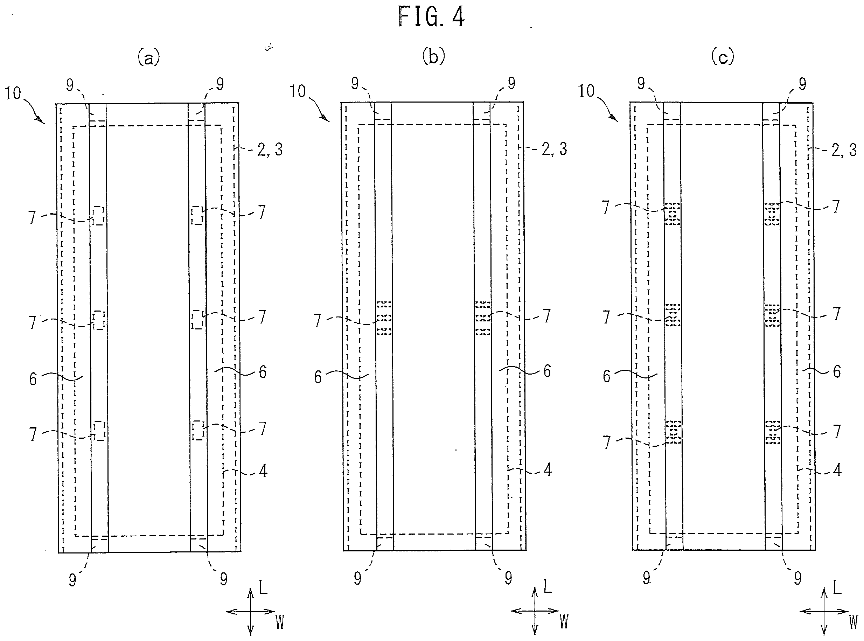

FIG. 4 is a plan view which shows a modification example of the configuration of the absorbent main body portion 10 of the disposable diaper 1. The configuration of the modification example of FIG. 4(a) arranges the temporarily fixed portion 7a and / or 7b at the equally divided portions when the leakage prevention portions 6b, 6c are substantially equally divided into four in the longitudinal direction L. In this case, the size of one temporarily fixed portion 7 may be the same or smaller in comparison with the size of the temporarily fixed portion 7 in a case in which the side sheet 6 has the configuration of FIG. 1. Further, the number of equally dividing the leakage prevention portions 6b, 6c of the side sheet 6 in the longitudinal direction L is not limited to four, and may be three or may be five or more. The configuration of the modification example of FIG. 4(b) includes a plurality of small temporarily fixed parts at the position of one temporarily fixed portion 7 in FIG. 1. In this case, the size of one temporarily fixed part is smaller in comparison with the size of the temporarily fixed portion 7 in a case in which the side sheet 6 has the configuration of FIG. 1. The number of the plurality of small temporarily fixed parts at the position of one temporarily fixed portion 7 is not particularly limited as long as they are two or more. The shape of the plurality of small temporarily fixed parts is not particularly limited, and for example, square, circle, rectangle, ellipse, etc., may be mentioned. The configuration of the modification example of FIG. 4(c) sets the positions of the equally divided points when the leakage prevention portions 6b, 6c of the side sheet 6 are substantially equally divided into four in the longitudinal direction L as the position of one temporarily fixed portion 7, and the position of the one temporarily fixed portion 7 includes the plurality of small temporarily fixed parts. That is, the configuration of FIG. 4(c) is a combination of the configuration of FIG. 4(a) and the configuration of FIG. 4(b).

In the present embodiment, the disposable diaper 1 further includes a liquid impermeable cover sheet 5. In the present embodiment, the cover sheet 5 includes the cover sheet 5a positioned on the skin side and the cover sheet 5b positioned on the non - skin side, which are laminated with each other in the thickness direction T and are joined by an adhesive agent, etc. In the present embodiment, the both end portions in the longitudinal direction L of the cover sheet 5b are folded back to the skin side so as to cover the both end portions in the longitudinal direction L of the cover sheet 5a. The cover sheets 5b, 5b at the folded back positions in the ventral side portion 11 and the dorsal side portion 13 configure the end portion 11e of the ventral side portion 11, and the end portion 13e of the dorsal side portion 13, respectively. On the skin side surface of the cover sheet 5, the absorbent main body portion 10 is arranged in a state in which the top sheet 2 faces the skin side. In the disposable diaper 1, the non - skin side surface of the disposable diaper 1, that is, the outer surface, is formed by the cover sheet 5b, and the skin side surface of the disposable diaper 1, that is, the inner surface, is formed by the top sheet 2, and the cover sheet 5b of the end portion 11e and the end portion 13e. As the cover sheet 5, for example, any liquid impermeable sheet such as a liquid impermeable nonwoven fabric, a synthetic resin film, a composite sheet of a nonwoven fabric and a synthetic resin film, an SB nonwoven fabric, an SMS nonwoven fabric, etc., may be mentioned. As the material of the cover sheet 5, for example, polyolefin - based materials such as polypropylene, polyethylene, etc., may be mentioned. The basis weight of the cover sheet 5 is, for example, 5 to 100 g / m², and is preferably 10 to 50 g / m². The dimension (the thickness) in the thickness direction T of the cover sheet 5 is, for example, 0.2 to 5 mm, and is preferably 0.2 to 2 mm. Incidentally, the cover sheet 5 may be one sheet, and may not be folded back.

In the present embodiment, the disposable diaper 1 further includes the elastic member 8 (8a, 8b, 8c, and 8d). The elastic member 8a and the elastic member 8b extend along the width direction W, are arranged with an interval in the longitudinal direction L, and are sandwiched, between the cover sheet 5a and the cover sheet 5b, in the ventral side portion 11 and the dorsal side portion 13, respectively. The elastic members 8a, 8b make the waist opening portion WO shrink. The elastic member 8c is continuously arranged so as to substantially be along the longitudinal direction L at the both end portions in the width direction W at the portion on the dorsal side portion 13 side in the crotch portion 12, and so as to be along the width direction W at the center portion in the crotch portion 12. The elastic member 8c makes the pair of the leg opening portions LO, LO shrink. At the end portion on the inner side in the width direction W of the leakage prevention portion 6b and the leakage prevention portion 6c in each of the side sheets 6, the elastic member 8d which extends along the longitudinal direction L is arranged. For example, one elastic member 8d is arranged in the leakage prevention portion 6b, and three elastic members 8d are arranged in the leakage prevention portion 6c, whereby make the leakage prevention portions 6b, 6c shrink in the longitudinal direction L. As the elastic member 8, for example, a thread rubber may be mentioned.

Incidentally, the absorbent main body portion 10 may include one or a plurality of other liquid permeable sheets between the top sheet 2 and the absorbent body 4. For example, a known sheet which gives the liquid diffusivity and cushioning property to the absorbent main body portion 10 may be mentioned. Further, tissue may be included between the back sheet 3 and the absorbent body 4 so as to replenish the absorbency performance.

Further, the absorbent main body portion 10 may include a pair of embossed portions (which are not shown) which extend along the longitudinal direction L, are arranged with an interval in the width direction W, and have a shape which are recessed from the skin side surface of the top sheet 2 to the non - skin side surface of the absorbent body 4 in the thickness direction T. Each of the embossed portions may have any planar shape, and for example, the same may have a gentle arc shape which is protruded or depressed with respect to the longitudinal direction center line CL, in a plan view.

In the present embodiment, the disposable diaper 1 (the hygiene product) includes the absorbent main body portion 10 (the functional member) which is formed by a plurality of sheet-like materials, that is, a sheet-like absorbent main body portion 10 (the functional member). Such absorbent main body portion 10 includes, on one surface in the thickness direction T, the pair of leakage prevention portions 6b, 6b and / or 6c, 6c (the pair of folded portions) which extend along the longitudinal direction L, are arranged side by side in the width direction W, and are folded in the thickness direction T. The region between the both end portions in the longitudinal direction L in each of the pair of leakage prevention portions 6b, 6b and / or 6c, 6c is set to be movable. Further, the disposable diaper 1 includes the temporarily fixed portion 7 which temporarily fixes the region between the both end portions in the longitudinal direction L in at least one of the pair of leakage prevention portions 6b, 6b and / or 6c, 6c.

In the disposable diaper 1 (the hygiene product), at least one of the leakage prevention portion 6b and / or 6c (the folded portion) is temporarily fixed after being manufactured until the beginning of usage, that is, the same is temporarily fixed to another portion. Accordingly, although the leakage prevention portion 6b and / or 6c is originally movable, since the same is temporarily fixed, the same cannot move even when vibration or force is applied from outside during the storage of the disposable diaper 1, and accordingly, it becomes difficult for the same to be deformed, for example, to turn over. Therefore, the leakage prevention portion 6b and / or 6c can suitably demonstrate the predetermined function with the beginning of usage, that is, the leakage prevention function by standing up on the skin side surface of the top sheet 2. Further, since the leakage prevention portion 6b and / or 6c is not deformed during the storage of the disposable diaper 1 by being temporarily fixed, the straight line portion of the fold is properly retained, and the disposable diaper 1 can maintain the appearance as a product with high quality.

Further, as a preferred aspect of the present embodiment, in the disposable diaper 1 (the hygiene product), at the region between both end portions in the longitudinal direction L in the leakage prevention portion 6b and / or 6c (the folded portion), when the one end portion in the width direction W is set to be the fixed end, and the other end portion is set to be the movable end, the temporarily fixed portion 7 is arranged in a region closer to the movable end than the fixed end. To be specific, in the example of FIG. 2, in the leakage prevention portion 6b, the temporarily fixed portion 7a is arranged in the region closer to the movable end (the end portion on the side which is connected to the leakage prevention portion 6c) than the fixed end in the width direction W (the end portion on the side which is connected to the covered portion 6a). In this case, in the width direction W, the position of the center of the temporarily fixed portion 7a is positioned on the movable end side in comparison with the center of the leakage prevention portion 6b. In the same manner, in the leakage prevention portion 6c, the temporarily fixed portion 7b is arranged in the region closer to the movable end (the end portion on the side which is not connected) than the fixed end in the width direction W (the end portion on the side which is connected to the leakage prevention portion 6b). In this case, in the width direction W, the position of the center of the temporarily fixed portion 7b is positioned on the movable end side in comparison with the center of the leakage prevention portion 6c. Further, also in the example of FIG. 3, the same can be applied with the leakage prevention portion 6b in the example of FIG. 2.

In the disposable diaper 1 (the hygiene product), the temporarily fixed portion 7a and / or 7b is formed in the region close to the movable end in the leakage prevention portion 6b and / or 6c (the folded portion). It is easier for the region close to the movable end in the leakage prevention portion 6b and / or 6c to move and to be turned over in comparison with the region close to the fixed end. Accordingly, the temporarily fixed portion 7a and / or 7b is formed in such a region in which it is easy to move, whereby it becomes difficult for the movable end of the leakage prevention portion 6b and / or 6c to deform and to be turned over.

Especially, as a preferred aspect of the present embodiment, as shown in FIG. 3, in the width direction W, when the length of the leakage prevention portion 6b is set to d0, and the half length of the leakage prevention portion 6b is set to d1 ( = d0 / 2), the temporarily fixed portion 7 is arranged in the half region on the movable end side among the leakage prevention portion 6b, that is, the range of the length d1 from the edge on the movable end side. Accordingly, the temporarily fixed portion 7 is formed in a region in which it is even easier to move, whereby it becomes more difficult for the movable end of the leakage prevention portion 6b (the folded portion) to deform and to be turned over. In addition, the temporarily fixed portion 7 is not arranged in the half region on the fixed end side among the leakage prevention portion 6b. Accordingly, it becomes easier for the wearer to remove the temporarily fixed portion 7a and / or 7b at the beginning of usage. Also in the configuration shown in FIG. 2, in the width direction W, the temporarily fixed portion 7a may be arranged in the half region on the movable end side among the leakage prevention portion 6b and the temporarily fixed portion 7b may be arranged in the half region on the movable end side among the leakage prevention portion 6c, whereby the same effect can be obtained.

Next, one example of the manufacturing method of the disposable diaper 1 according to the present embodiment is explained.

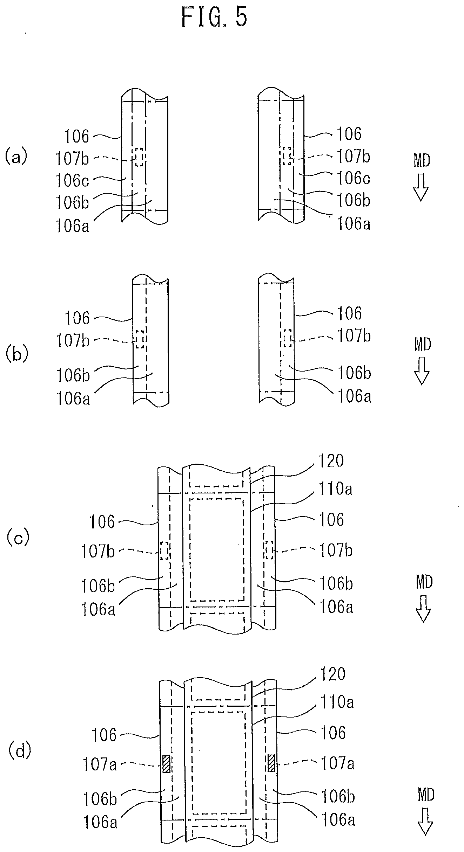

Next, one example of the manufacturing method of the disposable diaper 1 which includes the configuration as shown in FIG. 1 and FIG. 2 according to the present embodiment is explained. FIG. 5 to FIG. 7 are schematic views which show the manufacturing method of the disposable diaper 1 according to the embodiment. The manufacturing apparatus (which is not shown) which executes the manufacturing method includes the machine direction MD in which materials are conveyed, the cross machine direction CD which is vertical to the machine direction MD, and the transverse direction TD which is vertical to the machine direction MD and the cross machine direction CD. Hereinbelow, explanations are given in detail.

As shown in FIG. 5(a), the pair of continuous side sheet members 106, 106 which have a configuration in which a plurality of the pair of side sheets 6, 6 are connected in the longitudinal direction L is conveyed in the machine direction MD. In each of the continuous side sheet members 106, the portions which correspond to the covered portion 6a, the leakage prevention portion 6b, and the leakage prevention portion 6c of the absorbent main body portion 10 are the covered portion 106a, the leakage prevention portion 106b, and the leakage prevention portion 106c, respectively. Further, together with the conveyance, in each of the continuous side sheet members 106, an adhesive agent (for example: a hot melt adhesive agent) is applied to the position which corresponds to the temporarily fixed portion 7b of the absorbent main body portion 10. It should be noted that in FIG. 5(a), the adhesive agent is applied to the surface on the back side of the continuous side sheet members 106. Accordingly, the pair of temporarily fixed portions 107b, 107b are formed at positions which correspond to the pair of temporarily fixed portions 7b, 7b in the pair of continuous side sheet members 106, 106. Incidentally, in FIG. 5(a), the temporarily fixed portion 107b is formed on the surface on the back side of the continuous side sheet members 106.

Subsequently, as shown in FIG. 5(b), the pair of continuous side sheet members 106, 106 are conveyed in the machine direction MD. Further, together with the conveyance, the leakage prevention portion 106c in each of the continuous side sheet members 106 is folded along the folding line which extends in the machine direction MD so as to be folded toward the inner side in the cross machine direction CD, and is stacked on one surface in the transverse direction TD in the leakage prevention portion 106b. It should be noted that in FIG. 5(b), the pair of leakage prevention portions 106c are folded toward the direction of the back side of the continuous side sheet members 106. Accordingly, in the pair of continuous side sheet members 106, 106, the pair of leakage prevention portions 106c, 106c are temporarily fixed to the pair of leakage prevention portions 106b, 106b, by the pair of temporarily fixed portions 107b, 107b.

Subsequently, as shown in FIG. 5(c), by a conventionally known manufacturing method, the continuous sheet member 120 is formed and is conveyed in the machine direction MD. The continuous sheet member 120 has a configuration in which a plurality of the laminated bodies 110a for the lamination body 10a are connected in the machine direction MD so that the longitudinal direction L, the width direction W, and the thickness direction T of the lamination body 10a correspond to the machine direction MD, the cross machine direction CD, and the transverse direction TD, respectively. It should be noted that although not shown, each of the laminated bodies 110a of the continuous sheet member 120 includes the continuous top sheet member 102 for the top sheet, the continuous back sheet member 103 for the back sheet 3, and the absorbent body 104 of the absorbent body 4 (including the absorbent material 104a for the absorbent material 4a and the liquid permeable sheet 104b for the liquid permeable sheet 4b).

On the other hand, the pair of continuous side sheet members 106, 106 which have been subjected to the process of FIG. 5(b) is conveyed in the machine direction MD. At this time, an adhesive agent (for example : a hot melt adhesive agent; which is not shown) is applied to the upper side surface in the transverse direction TD of the inner side portion in the cross machine direction CD (the pair of covered portions 106a, 106a) in each of the pair of continuous side sheet members 106, 106. Further, together with the conveyance, the lower side surface in the transverse direction TD of the both end portions in the cross machine direction CD in the continuous sheet member 120 is overlapped with the upper side surface in the transverse direction TD (which is applied with an adhesive agent) of the inner side portion in the cross machine direction CD (the pair of covered portions 106a, 106a) in each of the pair of continuous side sheet members 106, 106. Accordingly, the both end portions in the cross machine direction CD in the continuous sheet member 120 are joined to the upper side surface in the transverse direction TD (which is applied with an adhesive agent) of the inner side portion in the cross machine direction CD (the pair of covered portions 106a, 106a) in each of the pair of continuous side sheet members 106, 106. At this time, the continuous sheet member 120 to which the pair of continuous side sheet members 106, 106 are joined can be regarded as having a configuration in which a plurality of the absorbent main body portions 110 are connected in the machine direction MD so that the longitudinal direction L, the width direction W, and the thickness direction T correspond to the machine direction MD, the cross machine direction CD, and the transverse direction TD, respectively.

Subsequently, as shown in FIG. 5(d), the continuous sheet member 120 to which the pair of continuous side sheet members 106, 106 are joined is conveyed in the machine direction MD. Further, together with the conveyance, in each of the continuous side sheet members 106, an adhesive agent (for example: a hot melt adhesive agent) is applied to the position which correspond to the temporarily fixed portion 7a of the absorbent main body portion 10. It should be noted that in FIG. 5(d), the adhesive agent is applied to the surface on the top side of the continuous side sheet members 106. Accordingly, the pair of temporarily fixed portions 107a, 107a are formed at positions which correspond to the pair of temporarily fixed portions 7a, 7a in the pair of continuous side sheet members 106, 106. Incidentally, in FIG. 5(d), the temporarily fixed portions107a are formed on the surface on the top side of the continuous side sheet members 106.

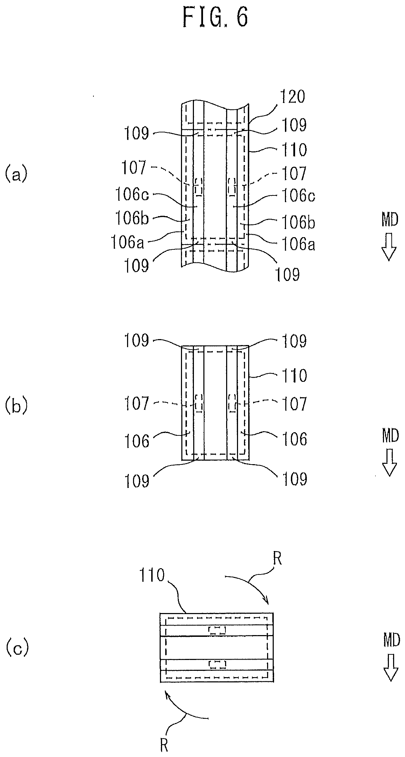

Subsequently, as shown in FIG. 6(a), the continuous sheet member 120 to which the pair of continuous side sheet members 106, 106 are joined is conveyed in the machine direction MD. Further, together with the conveyance, a portion of the leakage prevention portion 106b (including the leakage prevention portion 106c) and the covered portion 106a in each of the continuous side sheet members 106 is folded toward the inner side in the cross machine direction CD along the folding line which extends in the machine direction MD, and is stacked on one surface in the transverse direction TD in the continuous sheet member 120. As a result, in the laminated body 1 10a, while the pair of covered portions 106a, 106a are joined to the both end portions in the cross machine direction CD, the pair of leakage prevention portions 106b, 106b are temporarily fixed to (the continuous top sheet member 102 of) the laminated body 110a by the pair of temporarily fixed portions 107a, 107a. Accordingly, the configuration of each of the absorbent main body portions 110 is to be almost the same as the configuration of the absorbent main body portion 10. Further, at this time, or before or after the same, the fixed portions 9, 9 which fix the folded pair of leakage prevention portions 106b, 106b to the absorbent main body portion 110 are formed at both end portions in the machine direction MD in the folded pair of leakage prevention portions 106b, 106b and 106c, 106c, by methods such as heat sealing, ultrasonic sealing, or mechanical crimping.

It should be noted that the process from at least FIG. 5(d) to FIG. 6(a) can be regarded as the folded portion forming process in which the pair of folded portions which extend along the machine direction MD, are arranged side by side in the cross machine direction CD, and are folded in the transverse direction TD, that is, the folded pair of leakage prevention portions 106b, 106b, are formed on one surface in the transverse direction TD in the continuous sheet member 120, for each of the absorbent main body portions 110. Further, the process from at least FIG. 5(d) to FIG. 6(a) can be regarded as the temporarily fixing process in which the temporarily fixed portion 7a is formed in the region between the both end portions in the machine direction MD in at least one of the folded pair of leakage prevention portions 106b, for each of the absorbent main body portions 110 in the continuous sheet member 120. It should be noted that the folded portion forming process may include the process of FIG. 5(a) to FIG. 5(c). Further, the temporarily fixing process may include the process of FIG. 5(a) to FIG. 5(c). Accordingly, in the present embodiment, the folded portion forming process and the temporarily fixing process are performed at the same time.

Incidentally, as another embodiment, in a case in which the temporarily fixed portion does not use an adhesive agent, and uses fusion for example by ultrasonic sealing (or heat sealing), after the folded portion forming process, as the temporarily fixing process, the predetermined portions in the leakage prevention portion are fused to the continuous sheet member by an ultrasonic horn (a heat roll in the case of heat sealing) at the position of the temporarily fixed portion. In such a case, after the continuous sheet member in which the plurality of functional members are connected in the machine direction is formed by the folded portion forming process, the temporarily fixing process is performed.

Subsequently, as shown in FIG. 6(b), while conveying the continuous sheet member 120 to which the pair of continuous side sheet members 106, 106 are joined in the machine direction MD, the continuous sheet member 120 is cut along the cross machine direction CD for each of the absorbent main body portions 110 (the cutting process). Accordingly, the individually cut the absorbent main body portion 110 is substantially the absorbent main body portion 10.

Subsequently, as shown in FIG. 6(c), the individually cut the absorbent main body portion 110 is rotated as shown in R in the drawing, so that the width direction W corresponds to the machine direction MD, that is, the same is rotated by 90° (the rotating process). The rotation apparatus which rotates the absorbent main body portion 110 is not particularly limited, and for example, the apparatus described in Japanese Unexamined Patent Publication No. 2012 - 11048 (US20130140755(A1)), and the apparatus described in Japanese Unexamined Patent Publication No. 2015 - 510832 (WO2013/148380(A1)) may be mentioned. Accordingly, the pair of leakage prevention portions 106b, 106b and 106c, 106c of the absorbent main body portion 110 extend in the cross machine direction CD, whereby the same is brought to be in a state in which it is relatively easy to receive wind pressure while being conveyed in the machine direction MD. It should be noted that since each of the leakage prevention portions 106b, 106b is temporarily fixed to each of the temporarily fixed portions 107a, 107a, it is difficult for the same to move by the wind pressure, for example to be turned over. Further, the absorbent main body portion 110 is conveyed in the machine direction MD while the opposite side surface of the surface in which the pair of leakage prevention portions 106b, 106b and 106c, 106c are formed in the rotated absorbent main body portion 110 is set as the conveying surface (the conveying process). It should be noted that the conveying surface is the surface which comes into contact with the absorbent main body portion 110 in the conveying apparatus which conveys the absorbent main body portion 110.

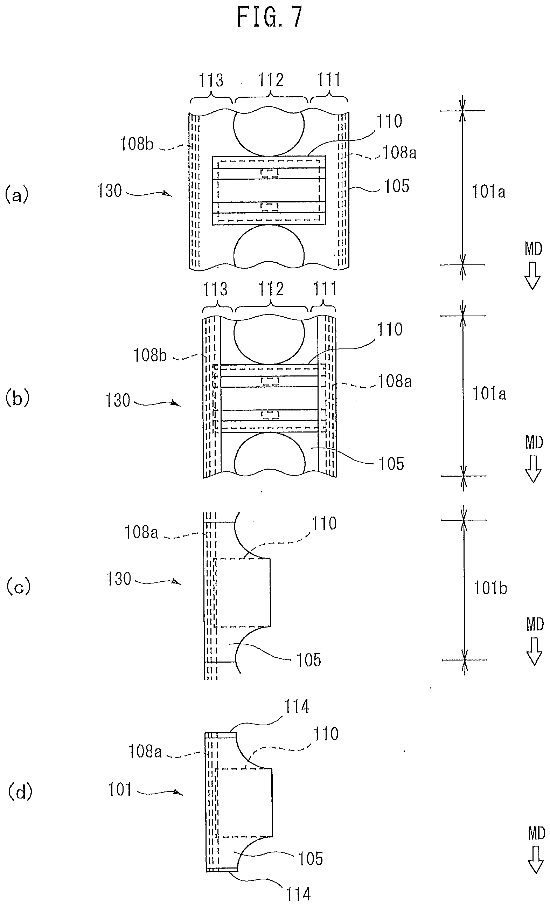

Subsequently, as shown in FIG. 7(a), by a conventionally known manufacturing method, the continuous sheet member 130 is formed and is conveyed in the machine direction MD. The continuous sheet member 130 includes the ventral side continuous portion 111 in which a plurality of ventral side portions 11 are connected in the width direction W (the machine direction MD) and the dorsal side continuous portion 113 in which a plurality of dorsal side portions 13 are connected in the width direction W (the machine direction MD), in both sides in the longitudinal direction L (the cross machine direction CD). Incidentally, the continuous sheet member 130 includes, between the ventral side continuous portion 111 and the dorsal side continuous portion 113, the intermediate continuous portion 112 in which a plurality of crotch portions 12 are connected in the width direction W (the machine direction MD). On the ventral side continuous portion 111 side in the cross machine direction CD of the continuous sheet member 130, inside the continuous sheet member 130, the elastic member 108a for the elastic member 8a is included, and on the dorsal side continuous portion 113 side, the elastic member 108b for the elastic member 8b is included. Further, inside the continuous sheet member 130, the elastic member (which is not shown) for the elastic members 8c, 8c is arranged.

Further, together with the conveyance, in the continuous sheet member 130, the absorbent main body portion 110 is arranged on the ventral side portion 11, the crotch portion 12, and the dorsal side portion 13 which are arranged side by side in the longitudinal direction L (the cross machine direction CD). Accordingly, the precursor 101a of the disposable diaper 1 (FIG. 2) in which the ventral side portion 11, the crotch portion 12, and the dorsal side portion 13 which are arranged side by side in the cross machine direction CD, and the absorbent main body portion 110 are integrated is formed. That is, the continuous sheet member 130 can be regarded as having a configuration in which the precursors 101a of the disposable diapers 1 in an expanded state (FIG. 2) are connected in the width direction W (the machine direction MD).

Subsequently, as shown in FIG. 7(b), while conveying the continuous sheet member 130 in the machine direction MD, the end portion of the ventral side continuous portion 111 and the end portion of the dorsal side continuous portion 113 in the cross machine direction CD are folded back so as to cover the both end portions in the cross machine direction CD of the absorbent main body portion 110.

Subsequently, as shown in FIG. 7(c), the continuous sheet member 130 is conveyed in the machine direction MD. Further, together with the conveyance, in the continuous sheet member 130, one of the ventral side continuous portion 111 and the dorsal side continuous portion 113 is folded onto the other so as to laminate the ventral side portion 11 and the dorsal side portion 13 (the folding process). In the present embodiment, the ventral side continuous portion 111 is folded and stacked onto the dorsal side continuous portion 113. As the folding apparatus so as to fold the continuous sheet member 130, for example, the apparatus described in Japanese Unexamined Patent Publication No. 2010 - 227545 (US20100179042(A1)), and the apparatus described in Japanese Unexamined Patent Publication No. 2007 - 125407 (US20020174930(A1) may be mentioned. In either methods, the absorbent main body portion 110 on the continuous sheet member 130 is folded while coming in contact with a guide roll or an apparatus member such as two-fold sailor. Accordingly, the precursors 101b of the disposable diaper 1 in which the ventral side portion 11 and the dorsal side portion 13 are overlapped are continuously formed. That is, the continuous sheet member 130 in which the precursors 101b of the disposable diaper 1 are continued in the machine direction MD is formed. It should be noted that the dorsal side continuous portion 113 may be overlapped on the ventral side continuous portion 111.

Subsequently, as shown in FIG. 7(d), the pair of joining portions 114, 114 which correspond to the pair of joining portions 14, 14 of the disposable diaper 1 are formed in the region across two precursors 101b, 101b adjacent in the machine direction MD (the joining process). As the joining method, for example, an adhesive agent, heat sealing, ultrasonic sealing, may be mentioned. Thereafter, in the continuous sheet member 130, the precursor 101b on the downstream side is cut along the boundary line which extends along the cross machine direction CD between the two adjacent precursors 101b, 101b. That is, the two adjacent precursors 101b, 101b are cut from each other along the boundary line between the adjacent joining portions 114, 114. Accordingly, the precursors 101b are individually separated, whereby the disposable diaper 101, that is, the disposable diaper 1 is manufactured.

In the manufacturing method of the disposable diaper 1 (the hygiene product) according to the present embodiment, before the absorbent main body portion 110 is rotated so that the width direction W corresponds to the machine direction MD, that is before the absorbent main body portion 110 is rotated by 90° (before the rotating process), the region between the both end portions in the longitudinal direction L (the machine direction MD) in at least one of the pair of leakage prevention portions 106b, 106b and / or 106c, 106c (the pair of folded portion) is temporarily fixed by the temporarily fixed portions 107a, 107a and / or 107b, 107b. As a result, in at least one of the pair of leakage prevention portions 106b, 106b and / or 106c, 106c, the region between the both end portions in the longitudinal direction L in which it is easy to move (the region of the movable end) is temporarily fixed, whereby even when the same is brought to a state in which it is relatively easy to receive wind pressure during conveyance, after the process of rotation by 90°, it becomes difficult to move, for example, to be turned over. Accordingly, at least one of the pair of leakage prevention portion 106b, 106b and / or 106c, 106c can be suppressed from, for example, being turned over in an unexpected direction by the wind pressure. That is, each of the leakage prevention portions 106b and / or 106c can be suppressed from being deviated from the planned position or shape due to the folding and joining in the processes in the later stage, for example, in the process of folding the absorbent main body portion 110, and the process of joining the members which include the absorbent main body portion 110 with each other, etc. Therefore, cases in which products cannot be shipped due to the disposable diapers 1 being defective products, or cases in which the appearance of the products when being used may be deteriorated can be suppressed. Incidentally, the wearer can easily release the temporarily fixed portion 7a and / or 7b (= the temporarily fixed portion 107a and / or 107b) when using the disposable diapers 1.

As a preferred aspect of the present embodiment, the above-mentioned temporarily fixing process includes a process of forming, in the machine direction MD, the temporarily fixed portion 107a and / or 107b in a region between fixed portions 109, 109 which fix the leakage prevention portion 106b and / or 106c (the folded portions) to the absorbent main body portion 110, positioned on the both end portions in at least one of the pair of leakage prevention portions 106b, 106b and / or 106c, 106c (the pair of folded portions).

In the present manufacturing method, in the machine direction MD, that is, in the longitudinal direction L of the absorbent main body portion 110, a region between the fixed portions 109 which are positioned on the both end portions in the leakage prevention portion 106b and / or 106c is temporarily fixed by the temporarily fixed portion 107a and / or 107b. Accordingly, it becomes difficult in a greater degree for the movable portion in the leakage prevention portion 106b and / or 106c to move across the entire longitudinal direction L of the absorbent main body portion 110, whereby it becomes more difficult for the same to be turned over.

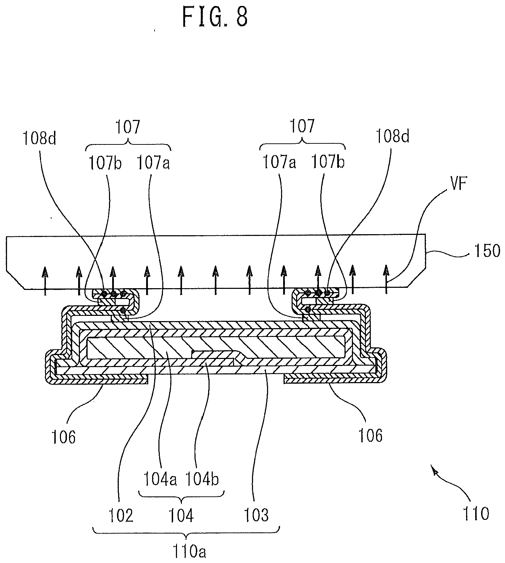

As a preferred aspect of the present embodiment, the above-mentioned rotating process includes a process of rotating the absorbent main body portion 110, while retaining the absorbent main body portion 110 by using an adsorption means by making the surface on which the pair of leakage prevention portions 106b, 106b and / or 106c, 106c (the pair of folded portions) are positioned in the absorbent main body portion 110 be adsorbed by the adsorption means of a rotation apparatus, by rotating the adsorption means. FIG. 8 is a schematic view which shows the adsorption state of the absorbent main body portion 110 in the rotating process of the manufacturing method of the disposable diaper according to the embodiment. As shown in FIG. 8, the surface on which the leakage prevention portions 106b and / or 106c are positioned in the absorbent main body portion 110 (the surface on the upper side in the absorbent main body portion 110 of FIG. 8) is adsorbed by the adsorption means 150 (for example: a retaining pad). That is, the surface on which the temporarily fixed portion 107a and / or 107b is positioned in the absorbent main body portion 110 is adsorbed by the suction of the atmosphere gas VF by the adsorption means 150. Further, by the rotation of the adsorption means 150, the absorbent main body portion 110 rotates.

In the rotating process, it is easy for the leakage prevention portion 106b and / or 106c (the folded portion) to move and to be turned over by the centrifugal force and the wind pressure at the time of rotation. That is, even when the entire absorbent main body portions 110 are moving in the predetermined speed and the turning over has not occurred in the leakage prevention portion 106b and / or 106c, in a case in which the machine direction MD is rotated by 90°, since the leakage prevention portion 106b and / or 106c is displaced in the direction of the wind while increasing the speed in accordance with the rotation, it is easier for the turning over to occur compared to the state of being conveyed before and after the rotation. Accordingly, in the present manufacturing method, after the leakage prevention portion 106b and / or 106c is temporarily fixed by the temporarily fixed portion 107a and / or 107b, the surface on which the leakage prevention portion 106b and / or 106c is positioned in the absorbent main body portion 110 is adsorbed by the adsorption means 150, so as to be rotated while retaining the absorbent main body portion 110. Accordingly, in the present manufacturing method, it becomes difficult for the leakage prevention portion 106b and / or 106c to move and the turning over to occur by the temporal fixing and the adsorption in the leakage prevention portion 106b and / or 106c, even during the rotating process.

Further, when the surface on which the leakage prevention portion 106b and / or 106c is positioned in the absorbent main body portion 110 is separated from the adsorption means 150, there may be cases in which the leakage prevention portion 106b and / or 106c is pulled by the adsorption means 150, so that the leakage prevention portion 106b and / or 106c moves and the turning over occurs. However, in the present manufacturing method, since the leakage prevention portion 106b and / or 106c is temporarily fixed, even when the leakage prevention portion 106b and / or 106c is pulled by the adsorption means 150, the leakage prevention portion 106b and / or 106c can be suppressed from being moved and the turning over can be suppressed from occurring.

As a preferred aspect of the present embodiment, the present manufacturing method further includes a folding process, after the conveying process, while making the surface on which the pair of leakage prevention portions 106b, 106b and /or 106c, 106c (the pair of folded portions) are positioned in the absorbent main body portion 110 in contact with a folding means (for example: a guide roll or a two-fold sailor) of a folding apparatus, of folding the surface along a folding line along the machine direction MD.

In the folding process, the absorbent main body portion 110 is folded while the folding means of the folding apparatus is in contact with the pair of leakage prevention portion 106b, 106b and / or 106c, 106c, whereby it is easy for the leakage prevention portion 106b and / or 106c (the folded portion) to move and the turning over to occur. However, in the present manufacturing method, since the leakage prevention portion 106b and / or 106c (the folded portion) is temporarily fixed by the temporarily fixed portion 107a and / or 107b, it becomes difficult for the leakage prevention portion 106b and / or 106c to move and for the turning over to occur even in such cases.

As a preferred aspect of the present embodiment, in the region between the both end portions in the longitudinal direction L (the machine direction MD) of at least one of the pair of leakage prevention portions 106b, 106b and / or 106c, 106c (the pair of folded portions), one end portion in the width direction W (the cross machine direction CD) is a fixed end, and the other end portion is a movable end, and the temporarily fixing process includes a process of forming the temporarily fixed portion 107a and / or 107b in a region closer to the movable end than the fixed end.

It is easier for the region close to the movable end in the leakage prevention portion 106b and / or 106c (the folded portion) to move and to turn over, compared to the region close to the fixed end, the temporarily fixed portion is formed in such a region. Accordingly, it becomes difficult for the movable portion of the leakage prevention portion 106b and / or 106c to move, and for the turning over to occur by forming the temporarily fixed portion 107a and / or 107b in such a region.

Incidentally, as another embodiment, in a case in which the temporarily fixed portion 107a is formed in only one of the pair of leakage prevention portions 106b, 106b, after the rotation of the rotating process, the absorbent main body portion 110 is rotated by the rotating process, or the temporarily fixed portion 107a is formed by the temporarily fixing process, so that the leakage prevention portion 106b which includes the temporarily fixed portion 107a is to be on the upstream side and the leakage prevention portion 106b which does not include the temporarily fixed portion 107a is to be on the downstream side. Alternatively, as still another embodiment, in a case in which the temporarily fixed portion 107b is formed in only one of the pair of leakage prevention portions 106c, 106c, after the rotation of the rotating process, the absorbent main body portion 110 is rotated by the rotating process, or the temporarily fixed portion 107b is formed by the temporarily fixing process, so that the leakage prevention portion 106c which includes the temporarily fixed portion 107b is to be on the downstream side and the leakage prevention portion 106c which does not include the temporarily fixed portion 107b is to be on the upstream side. Alternatively, as still another embodiment, in a case in which the temporarily fixed portion 107a is formed in only one of the pair of leakage prevention portions 106b, 106b, and the temporarily fixed portion 107b is formed in only one of the pair of leakage prevention portions 106c, 106c, after the rotation of the rotating process, the absorbent main body portion 110 is rotated by the rotating process, or the temporarily fixed portions 107a, 107b are formed by the temporarily fixing process, so that the leakage prevention portion 106b which includes the temporarily fixed portion 107a is to be on the upstream side and the leakage prevention portion 106c which includes the temporarily fixed portion 107b is to be on the downstream side. In these cases, since the temporarily fixed portion can be reduced, in the manufacturing method, the temporarily fixing process can be simplified and further, in the products, the releasing of the temporarily fixed portion at the beginning of usage can be performed more easily and reliably.

Next, as another embodiment, explanation is given by mentioning a mask which is one of medical articles as an example of a hygiene product.

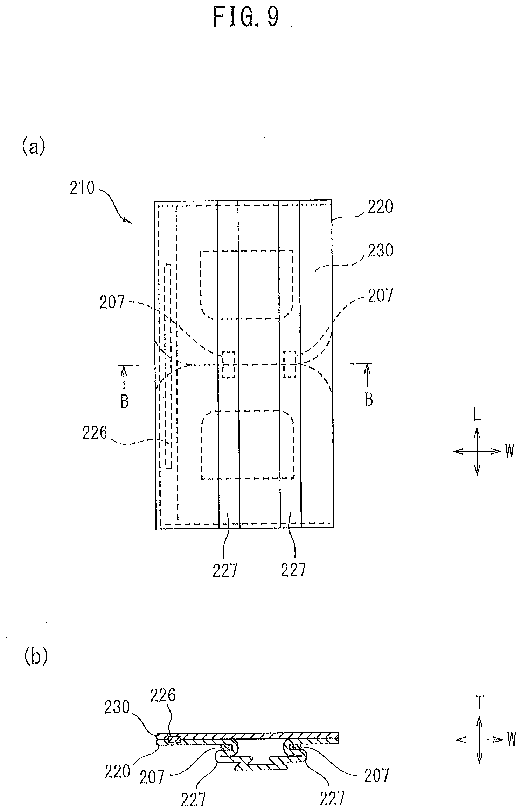

FIG. 9 is a view which shows the configurational example of the mask 210 according to the present embodiment. It should be noted that FIG. 9(a) is a plan view which shows the mask 210, and FIG. 9(b) is a cross sectional view along the B - B line of FIG. 9(a). The mask 210 includes the mask main body 220 (the functional member) which includes a single or a plurality of pieces of sheet-like nonwoven fabric including a filter sheet, and the pair of ear hook portions 230, 230. The mask main body 220 has a rectangular shape which is long in the longitudinal direction L, and includes the pair folds 227, 227 (the pair of folded portions) which extend along the longitudinal direction L, are arranged side by side in the width direction W, and are folded in the thickness direction T. In each of the pair folds 227, 227, the region between the both end portions in the longitudinal direction L is set to be movable, and deforms into a bellows shape so as to form a predetermined mouth space between the inner surface of the mask main body 220 and the wearer. The mask main body 220 further includes the temporarily fixed portion 207 which temporarily fixes the region between the both end portions in the longitudinal direction L in at least one of the pair folds 227, 227. In the present embodiment, both of the pair folds 227, 227 includes the temporarily fixed portion 207. Each of the pair of ear hook portions 230, 230 is arranged adjacent to each other, and the end portion on the opposite side of the adjacent side in the longitudinal direction L is joined to the end portion in the longitudinal direction L of the mask main body 220. In the present embodiment, the mask main body 220 includes the nose fit member 226, and further, a fixed portion (which is not shown) which fixes the folds 227 to the mask main body 220 may be formed at both end portions in the longitudinal direction L in at least one of the pair folds 227, 227.

Also in the mask 210, at least one of the folds 227 (the folded portions) is temporarily fixed by the temporarily fixed portion 207 after the manufacturing until the usage, that is, the same is temporarily fixed to another portion. Accordingly, at least the temporarily fixed folds 227 are not deformed during the storage of the mask 210, and can demonstrate the function of deforming into a bellows shape so as to form the mouth space between the inner surface of the mask main body 220 and the wearer properly from the beginning of usage. Further, at least one of the folds 227 are temporarily fixed, whereby the temporarily fixed folds 227 are not deformed during the storage of the mask 210, and thus the straight line portion of the fold is properly retained, and can maintain the appearance as a product with high quality.

The manufacturing method of the mask 210 may be performed for example in the following method.

First, the continuous sheet member is prepared in which the plurality of mask main bodies 220 are connected in the machine direction so that the longitudinal direction L, the width direction W, and the thickness direction T of the flat mask main body 220 which does not include the folds 227 correspond to the machine direction, the cross machine direction, and the transverse direction, respectively of the manufacturing apparatus. Subsequently, while conveying the flat continuous sheet member which does not include the folds 227 in the machine direction, the pair of folds 227 (the pair of folded portions) which extend along the machine direction, are arranged side by side in the cross machine direction and are folded in the transverse direction, are formed on one surface in the transverse direction in the continuous sheet member, for each of the mask main bodies 220 (the folded portion forming process).

Subsequently, for each of the mask main bodies 220 in the continuous sheet member, the temporarily fixed portion 207 is formed in the region between the both end portions in the machine direction in at least one of the pair of folds 227 (the pair of folded portions) (the temporarily fixing process). As the forming method of the temporarily fixed portion 207, for example, an ultrasonic sealing (or a heat sealing) which uses an ultrasonic horn (or a heat roll) may be applied.

Subsequently, the continuous sheet member is cut along the cross machine direction for each of the temporarily fixed mask main bodies 220 (the cutting process). Subsequently, the cut mask main body 220 is rotated so that the width direction W corresponds to the machine direction, that is, the same is rotated by 90° (the rotating process). Subsequently, the mask main body 220 is conveyed in the machine direction by setting the surface on the opposite side of the surface in which the pair of folds 227 (the pair of folded portions) are formed in the rotated mask main body 220 as the conveying surface (the conveying process).

Thereafter, the pair of ear hook portions 230, 230 which have been manufactured by another process and the mask main body 220 are joined, whereby the mask 210 is manufactured. Incidentally, the specific methods of the folded portion forming process, the cutting process, the rotating process, and the conveying process are as described in Patent Literature 2.

Also in the present embodiment, the same effect as the manufacturing method of the disposable diaper 1 as shown in FIG. 5 to FIG. 7 can be exhibited. That is, since in at least one of the pair of folds 227 (the pair of folded portions), the region between the both end portions in the longitudinal direction in which it is easy to move, is temporarily fixed, even when the same is brought to a state in which it is relatively easy to receive wind pressure during conveyance, after the process of rotation by 90°, it becomes difficult to move, for example, to be turned over. Accordingly, at least one of the pair of folds 227 can be suppressed from, for example, being turned over in an unexpected direction by the wind pressure. That is, each of the folds 227 (the folded portions) can be suppressed from being deviated from the planned position or shape due to the joining in the processes in the later stage, for example, in the process of joining the members which include the mask main body 220 with each other, etc. Therefore, cases in which products cannot be shipped due to the mask 210 (the hygiene product) being defective products, or cases in which the appearance of the products when being used may be deteriorated can be suppressed. Incidentally, the wearer can easily release the temporarily fixed portion 207 at the time of usage.

### REFERENCE SIGNS LIST

- 1: disposable diaper (hygiene product)
- 110: absorbent main body portion (functional member)
- 120: continuous sheet member
- 106b: folded portion
- 107a: temporarily fixed portion

## Claims

1. A manufacturing method of a hygiene product (1) which includes a sheet-like functional member (110), wherein the functional member (110) includes on one surface in a thickness direction, a pair of folded portions (106b, 106b) which extend along a longitudinal direction, are arranged side by side in a width direction, and are folded in the thickness direction, and a region between both end portions in the longitudinal direction in each of the pair of folded portions (106b, 106b) is movable, the manufacturing method comprising:
a folded portion forming process, while conveying in a machine direction, a continuous sheet member (120) in which the plurality of functional members (110) are connected in the machine direction so that the longitudinal direction, the width direction, and the thickness direction correspond to the machine direction, a cross machine direction, and a transverse direction, respectively of a manufacturing apparatus, of forming on one surface in the transverse direction of the continuous sheet member (120), the pair of folded portions (106b, 106b) which extend along the machine direction, are arranged side by side in the cross machine direction, and are folded in the transverse direction, for each of the functional members (110),
a temporarily fixing process of forming a temporarily fixed portion (107a) in a region between both end portions in the machine direction in at least one of the pair of folded portions (106b, 106b), for each of the functional members (110) of the continuous sheet member (120),
a cutting process of cutting the continuous sheet member (120) along the cross machine direction for each of the temporarily fixed functional members (110),
a rotating process of rotating each of the cut functional members (110) so that the width direction corresponds to the machine direction, and
a conveying process of conveying each of the rotated functional members (110) in the machine direction, in a state in which a surface on an opposite side of a surface on which the pair of folded portions (106b, 106b) are formed in each of the rotated functional members (110) is set as a conveying surface, wherein
the temporarily fixed portion (107a) is formed of an adhesive agent,
the temporarily fixed portion (107a) is formed so as to be removed at the time of usage of the hygiene product (1), and
the temporarily fixing process includes a process of forming the temporarily fixed portion (107a) only at a center portion of the region between both end portions in the machine direction in at least one of the pair of folded portions (106b, 106b), for each of the functional members (110) of the continuous sheet member (120),
the temporarily fixing process includes a process of forming, in the machine direction, the temporarily fixed portion (107a, 107b) in a region between fixed portions which fix at least one of the pair of the folded portions (106b, 106c, 106b, 106c) to each of the functional members (110), positioned on the both end portions in the at least one of the pair of folded portions (106b, 106c, 106b, 106c),
the at least one of the pair of folded portions (106b, 106c, 106b, 106c) includes a lower folded portion (106b) and an upper folded portion (106c) which is folded on the lower folded portion (106b) in the thickness direction, and
the temporarily fixed portion (107a, 107b) is formed between each of the functional members (110) and the lower folded portion (106a), and between the lower folded portion (106b) and the upper folded portion (106c) in the region between fixed portions, and wherein
the hygiene product (1) is an absorbent article,
the functional member (110) is an absorbent main body portion which includes a top sheet, a back sheet, and an absorbent body which is positioned between the top sheet and the back sheet, and
the pair of folded portions (106b, 106b) are a pair of leakage prevention walls.

2. The manufacturing method of the hygiene product according to claim 1, wherein
the rotating process includes a process of rotating each of the functional members (110), while retaining each of the functional members (110) using an adsorption means by making a surface on which the pair of folded portions (106b, 106b) are positioned in each of the functional members (110) be adsorbed by the adsorption means of a rotation apparatus, by rotating the adsorption means.

3. The manufacturing method of the hygiene product according to claim 1 or 2, further comprising a folding process, after the conveying process, while making a surface on which the pair of folded portions (106b, 106b) are positioned in each of the functional members (110) in contact with a folding means of a folding apparatus, of folding the surface along a folding line along the machine direction.

4. The manufacturing method of the hygiene product according to any one of claims 1 to 3, wherein
in a region between both end portions in the longitudinal direction of at least one of the pair of folded portions (106b, 106b), one end portion in the cross machine direction is a fixed end, and the other end portion is a movable end, and
the temporarily fixing process includes a process of forming the temporarily fixed portion (107a) in a region closer to the movable end than the fixed end.

5. A hygiene product (1) which includes a sheet-like functional member, wherein the functional member includes on one surface in a thickness direction, a pair of folded portions (106b, 106b) which extend along a longitudinal direction, are arranged side by side in a width direction, and are folded in the thickness direction, and a region between both end portions in the longitudinal direction in each of the pair of folded portions (106b, 106b) is movable, comprising:
a temporarily fixed portion (107a) which temporarily fixes a region between both end portions in the longitudinal direction in at least one of the pair of folded portions (106b, 106b), wherein the temporarily fixed portion (107a) is formed of an adhesive agent,
the temporarily fixed portion (107a) is configured to be removed at the time of usage of the hygiene product (1), and
the temporarily fixed portion (107a) is arranged only at a center portion of the region between both end portions in the longitudinal direction in at least one of the pair of folded portions (106b, 106b), for each of the functional members (110) of the continuous sheet member (120).
the temporarily fixed portion (107a, 107b) is arranged in a region between fixed portions which fix at least one of the pair of the folded portions (106b, 106c, 106b, 106c) to each of the functional members (110), positioned on the both end portions in the at least one of the pair of folded portions (106b, 106c, 106b, 106c),
the at least one of the pair of folded portions (106b, 106c, 106b, 106c) includes a lower folded portion (106b) and an upper folded portion (106c) which is folded on the lower folded portion (106b) in the thickness direction, and
the temporarily fixed portion (107a, 107b) is formed between each of the functional members (110) and the lower folded portion (106a), and between the lower folded portion (106a) and the upper folded portion (106c) in the region between fixed portions and wherein
the hygiene product (1) is an absorbent article,
the functional member (110) is an absorbent main body portion which includes a top sheet, a back sheet, and an absorbent body which is positioned between the top sheet and the back sheet, and
the pair of folded portions (106b, 106b) are a pair of leakage prevention walls.

## Patentansprüche

1. Herstellungsverfahren eines Hygieneprodukts (1), das ein lagenartiges funktionelles Element (110) beinhaltet, wobei das funktionelle Element (110) auf einer Fläche in einer Dickenrichtung ein Paar von gefalteten Abschnitten (106b, 106b) beinhaltet, das sich entlang einer Längsrichtung erstreckt, nebeneinander in einer Breitenrichtung angeordnet ist und in der Dickenrichtung gefaltet ist, und ein Bereich zwischen beiden Endabschnitten in der Längsrichtung in jedem des Paars von gefalteten Abschnitten (106b, 106b) beweglich ist, wobei das Herstellungsverfahren Folgendes umfasst:
einen Vorgang zum Bilden eines gefalteten Abschnitts, während in einer Maschinenrichtung ein durchgehendes Lagenelement (120) gefördert wird, bei dem die Vielzahl von funktionellen Elementen (110) in der Maschinenrichtung verbunden ist, sodass die Längsrichtung, die Breitenrichtung und die Dickenrichtung der Maschinenrichtung, einer Maschinenquerrichtung bzw. einer Querrichtung einer Herstellungsvorrichtung zum Formen auf einer Fläche in der Querrichtung des durchgehenden Lagenelements (120) entsprechen, wobei das Paar von gefalteten Abschnitten (106b, 106b), das sich entlang der Maschinenrichtung erstreckt, nebeneinander in der Maschinenquerrichtung angeordnet ist und für jedes funktionellen Elemente (110) in der Querrichtung gefaltet ist,
einen Vorgang zum vorübergehenden Fixierens zum Bilden eines vorübergehend fixierten Abschnitts (107a) in einem Bereich zwischen beiden Endabschnitten in der Maschinenrichtung in mindestens einem des Paars von gefalteten Abschnitten (106b, 106b) für jedes der funktionellen Elemente (110) des durchgehenden Lagenelements (120),
einen Schneidvorgang zum Schneiden des durchgehenden Lagenelements (120) entlang der Maschinenquerrichtung für jedes der vorübergehend fixierten funktionellen Elemente (110),
einen Drehvorgang zum Drehen jedes der geschnittenen funktionellen Elemente (110), sodass die Breitenrichtung der Maschinenrichtung entspricht, und
einen Fördervorgang zum Fördern jedes der rotierten funktionellen Elemente (110) in der Maschinenrichtung in einem Zustand, in dem eine Fläche auf einer gegenüberliegenden Seite einer Fläche, auf der das Paar von gefalteten Abschnitten (106b, 106b) in jedem der rotierten funktionellen Elemente (110) gebildet ist, als eine Förderfläche festgelegt ist, wobei
der vorübergehend fixierte Abschnitt (107a) aus einem Haftmittel gebildet ist,
der vorübergehend fixierte Abschnitt (107a) derart gebildet ist, dass er zum Zeitpunkt der Verwendung des Hygieneprodukts (1) entfernt wird, und
der Vorgang zum vorübergehenden Fixieren einen Vorgang zum Bilden des vorübergehend fixierten Abschnitts (107a) nur an einem mittleren Abschnitt des Bereichs zwischen beiden Endabschnitten in der Maschinenrichtung in mindestens einem des Paars von gefalteten Abschnitten (106b, 106b) für jedes der funktionellen Elemente (110) des durchgehenden Lagenelements (120) beinhaltet, wobei der Vorgang zum vorübergehenden Fixieren einen Vorgang zum Bilden des vorübergehend fixierten Abschnitts (107a, 107b) in der Maschinenrichtung in einem Bereich zwischen fixierten Abschnitten beinhaltet, der mindestens eines des Paars von gefalteten Abschnitten (106b, 106c, 106b, 106c) an jedem der funktionellen Elemente (110), die an den beiden Endabschnitten in dem mindestens einen des Paars von gefalteten Abschnitten (106b, 106c, 106b, 106c) positioniert sind, fixiert,
das mindestens eine des Paars von gefalteten Abschnitten (106b, 106c, 106b, 106c) einen unteren gefalteten Abschnitt (106b) und einen oberen gefalteten Abschnitt (106c) beinhaltet, der auf den unteren gefalteten Abschnitt (106b) in der Dickenrichtung gefaltet ist, und
der vorübergehend fixierte Abschnitt (107a, 107b) zwischen jedem der funktionellen Elemente (110) und dem unteren gefalteten Abschnitt (106a) und zwischen dem unteren gefalteten Abschnitt (106b) und dem oberen gefalteten Abschnitt (106c) in dem Bereich zwischen fixierten Abschnitten gebildet ist, und wobei
das Hygieneprodukt (1) ein absorbierender Artikel ist,
das funktionelle Element (110) ein absorbierender Hauptkörperabschnitt ist, der eine obere Lage, eine hintere Lage und einen absorbierenden Körper beinhaltet, der zwischen der oberen Lage und der hinteren Lage positioniert ist, und
das Paar von gefalteten Abschnitten (106b, 106b) ein Paar von Auslaufschutzwänden ist.

2. Herstellungsverfahren des Hygieneprodukts nach Anspruch 1, wobei
der Drehvorgang einen Vorgang zum Drehen jedes der funktionellen Elemente (110) beinhaltet, während jedes der funktionellen Elemente (110) unter Verwendung eines Adsorptionsmittels zurückgehalten wird, indem eine Fläche, auf der das Paar von gefalteten Abschnitten (106b, 106b) in jedem der funktionellen Elemente (110) positioniert ist, durch das Adsorptionsmittel einer Drehvorrichtung adsorbiert wird, indem das Adsorptionsmittel gedreht wird.

3. Herstellungsverfahren des Hygieneprodukts nach Anspruch 1 oder 2, ferner umfassend einen Faltvorgang nach dem Fördervorgang, während eine Fläche, auf der das Paar von gefalteten Abschnitten (106b, 106b) in jedem der funktionellen Elemente (110) in Kontakt mit einem Faltmittel einer Faltvorrichtung positioniert ist, zum Falten der Fläche entlang einer Faltlinie entlang der Maschinenrichtung.

4. Herstellungsverfahren des Hygieneprodukts nach einem der Ansprüche 1 bis 3, wobei
in einem Bereich zwischen beiden Endabschnitten in der Längsrichtung von mindestens einem des Paars von gefalteten Abschnitten (106b, 106b) ein Endabschnitt in der Maschinenquerrichtung ein fixiertes Ende ist und der andere Endabschnitt ein bewegliches Ende ist und
der Vorgang zum vorübergehenden Fixieren einen Vorgang zum Bilden des vorübergehend fixierten Abschnitts (107a) in einem Bereich näher an dem beweglichen Ende als an dem festen Ende beinhaltet.

5. Hygieneprodukt (1), das ein lagenartiges funktionelles Element beinhaltet, wobei das funktionelle Element auf einer Fläche in einer Dickenrichtung ein Paar von gefalteten Abschnitten (106b, 106b) beinhaltet, das sich entlang einer Längsrichtung erstreckt, nebeneinander in einer Breitenrichtung angeordnet ist und in der Dickenrichtung gefaltet ist, und ein Bereich zwischen beiden Endabschnitten in der Längsrichtung in jedem des Paars von gefalteten Abschnitten (106b, 106b) beweglich ist, umfassend:
einen vorübergehend fixierten Abschnitt (107a), der einen Bereich zwischen beiden Endabschnitten in der Längsrichtung in mindestens einem des Paars von gefalteten Abschnitten (106b, 106b) vorübergehend fixiert, wobei der vorübergehend fixierte Abschnitt (107a) aus einem Haftmittel gebildet ist,
der vorübergehend fixierte Abschnitt (107a) dazu konfiguriert ist, zum Zeitpunkt der Verwendung des Hygieneprodukts (1) entfernt zu werden, und
der vorübergehend fixierte Abschnitt (107a) nur an einem mittleren Abschnitt des Bereichs zwischen beiden Endabschnitten in der Längsrichtung in mindestens einem des Paars von gefalteten Abschnitten (106b, 106b) für jedes der funktionellen Elemente (110) des durchgehenden Lagenelements (120) angeordnet ist,
der vorübergehend fixierte Abschnitts (107a, 107b) in einem Bereich zwischen fixierten Abschnitten angeordnet ist, der mindestens eines des Paars von gefalteten Abschnitten (106b, 106c, 106b, 106c) an jedes der funktionellen Elemente (110), die an den beiden Endabschnitten in dem mindestens einen des Paars von gefalteten Abschnitten (106b, 106c, 106b, 106c) positioniert sind, fixiert,
das mindestens eine des Paars von gefalteten Abschnitten (106b, 106c, 106b, 106c) einen unteren gefalteten Abschnitt (106b) und einen oberen gefalteten Abschnitt (106c) beinhaltet, der auf den unteren gefalteten Abschnitt (106b) in der Dickenrichtung gefaltet ist, und
der vorübergehend fixierte Abschnitt (107a, 107b) zwischen jedem der funktionellen Elemente (110) und dem unteren gefalteten Abschnitt (106a) und zwischen dem unteren gefalteten Abschnitt (106a) und dem oberen gefalteten Abschnitt (106c) in dem Bereich zwischen fixierten Abschnitten gebildet ist, und wobei
das Hygieneprodukt (1) ein absorbierender Artikel ist,
das funktionelle Element (110) ein absorbierender Hauptkörperabschnitt ist, der eine obere Lage, eine hintere Lage und einen absorbierenden Körper beinhaltet, der zwischen der oberen Lage und der hinteren Lage positioniert ist, und
das Paar von gefalteten Abschnitten (106b, 106b) ein Paar von Auslaufschutzwänden ist.

## Revendications

1. Procédé de production d'un produit d'hygiène (1) qui comporte un élément fonctionnel en forme de feuille (110), dans lequel l'élément fonctionnel (110) comporte sur une surface dans le sens de l'épaisseur, une paire de parties pliées (106b, 106b) qui s'étendent le long d'une direction longitudinale, sont disposées côte à côte dans le sens de la largeur et sont pliées dans le sens de l'épaisseur, et une région entre les deux parties d'extrémité dans la direction longitudinale dans chacune de la paire de parties pliées (106b, 106b) est mobile, le procédé de fabrication comprenant :
un processus de formation de partie pliée, tout en transportant dans un sens machine, un élément en feuille continue (120) dans lequel la pluralité d'éléments fonctionnels (110) sont reliés dans le sens machine de sorte que la direction longitudinale, le sens de la largeur et le sens de l'épaisseur correspondent au sens machine, au sens transversal de la machine et à la direction transversale, respectivement d'un appareil de fabrication, de formation sur une surface dans la direction transversale de l'élément en feuille continue (120), de la paire de parties pliées (106b, 106b) qui s'étendent le long du sens machine, sont disposées côte à côte dans le sens transversal de la machine et sont pliées dans la direction transversale, pour chacun des éléments fonctionnels (110),
un processus de fixation temporaire destiné à former une partie temporairement fixée (107a) dans une région entre les deux parties d'extrémité dans le sens machine dans au moins l'une de la paire de parties pliées (106b, 106b), pour chacun des éléments fonctionnels (110) de l'élément en feuille continue (120),
un processus de découpe destiné à découper l'élément en feuille continue (120) le long du sens transversal de la machine pour chacun des éléments fonctionnels temporairement fixés (110),
un processus de rotation destiné à la rotation de chacun des éléments fonctionnels coupés (110) de sorte que le sens de la largeur correspond au sens machine, et
un processus de transport destiné à transporter chacun des éléments fonctionnels tournés (110) dans le sens machine, dans un état dans lequel une surface sur un côté opposé d'une surface sur laquelle la paire de parties pliées (106b, 106b) sont formées dans chacun des éléments fonctionnels tournés (110) est placée comme une surface de transport, dans lequel
la partie temporairement fixée (107a) est formée d'un agent adhésif,
la partie temporairement fixée (107a) est formée de manière à être retirée au moment de l'utilisation du produit d'hygiène (1), et
le processus de fixation temporaire comporte un processus de formation de la partie temporairement fixée (107a) uniquement au niveau d'une partie centrale de la région entre les deux parties d'extrémité dans le sens machine dans au moins l'une de la paire de parties pliées (106b, 106b), pour chacun des éléments fonctionnels (110) de l'élément en feuille continue (120), le processus de fixation temporaire comporte un processus de formation, dans le sens machine, de la partie temporairement fixée (107a, 107b) dans une région entre des parties fixes qui fixent au moins une de la paire de parties pliées (106b, 106c, 106b, 106c) à chacun des éléments fonctionnels (110), positionnée sur les deux parties d'extrémité dans l'au moins une de la paire de parties pliées (106b, 106c, 106b, 106c),
l'au moins une de la paire de parties pliées (106b, 106c, 106b, 106c) comporte une partie pliée inférieure (106b) et une partie pliée supérieure (106c) qui est pliée sur la partie pliée inférieure (106b) dans le sens de l'épaisseur, et
la partie temporairement fixée (107a, 107b) est formée entre chacun des éléments fonctionnels (110) et la partie pliée inférieure (106a), et entre la partie pliée inférieure (106b) et la partie pliée supérieure (106c) dans la région entre les parties fixes, et dans lequel
le produit d'hygiène (1) est un article absorbant,
l'élément fonctionnel (110) est une partie de corps principal absorbante qui comporte une feuille supérieure, une feuille arrière et un corps absorbant qui est positionné entre la feuille supérieure et la feuille arrière, et
la paire de parties pliées (106b, 106b) est une paire de parois anti-fuite.

2. Procédé de production d'un produit d'hygiène selon la revendication 1, dans lequel
le processus de rotation comporte un processus de rotation de chacun des éléments fonctionnels (110), tout en retenant chacun des éléments fonctionnels (110) à l'aide d'un moyen d'adsorption en faisant absorber une surface sur laquelle la paire de parties pliées (106b, 106b) sont positionnées dans chacun des éléments fonctionnels (110) par les moyens d'adsorption d'un appareil de rotation, en faisant tourner les moyens d'adsorption.

3. Procédé de fabrication du produit d'hygiène selon la revendication 1 ou 2, comprenant en outre un processus de pliage, après le processus de transport, tout en mettant en contact une surface sur laquelle la paire de parties pliées (106b, 106b) sont positionnées dans chacun des éléments fonctionnels (110) avec un moyen de pliage d'un appareil de pliage, de pliage de la surface le long d'une ligne de pliage dans le sens machine.

4. Procédé de production d'un produit d'hygiène selon l'une quelconque des revendications 1 à 3, dans lequel
dans une région entre les deux parties d'extrémité dans la direction longitudinale d'au moins l'une de la paire de parties pliées (106b, 106b), une partie d'extrémité dans le sens transversal de la machine est une extrémité fixe, et l'autre partie d'extrémité est une extrémité mobile, et
le processus de fixation temporaire comporte un processus de formation de la partie temporairement fixée (107a) dans une région plus proche de l'extrémité mobile que de l'extrémité fixe.

5. Produit d'hygiène (1) qui comporte un élément fonctionnel en forme de feuille, dans lequel l'élément fonctionnel comporte sur une surface dans le sens de l'épaisseur, une paire de parties pliées (106b, 106b) qui s'étendent le long d'une direction longitudinale, sont disposées côte à côte dans le sens de la largeur et sont pliées dans le sens de l'épaisseur, et une région entre les deux parties d'extrémité dans la direction longitudinale dans chacune de la paire de parties pliées (106b, 106b) est mobile, comprenant :
une partie temporairement fixée (107a) qui fixe temporairement une région entre les deux parties d'extrémité dans la direction longitudinale dans au moins l'une de la paire de parties pliées (106b, 106b), dans lequel la partie temporairement fixée (107a) est formée d'un agent adhésif,
la partie temporairement fixée (107a) est conçue pour être retirée au moment de l'utilisation du produit d'hygiène (1), et
la partie temporairement fixée (107a) est disposée uniquement au niveau d'une partie centrale de la région entre les deux parties d'extrémité dans la direction longitudinale dans au moins l'une de la paire de parties pliées (106b, 106b), pour chacun des éléments fonctionnels (110) de l'élément en feuille continue (120)
la partie temporairement fixée (107a, 107b) est disposée dans une région entre des parties fixes qui fixent au moins l'une de la paire des parties pliées (106b, 106c, 106b, 106c) à chacun des éléments fonctionnels (110), positionnées sur les deux parties d'extrémité dans l'au moins une de la paire de parties pliées (106b, 106c, 106b, 106c),
l'au moins une de la paire de parties pliées (106b, 106c, 106b, 106c) comporte une partie pliée inférieure (106b) et une partie pliée supérieure (106c) qui est pliée sur la partie pliée inférieure (106b) dans le sens de l'épaisseur, et
la partie temporairement fixée (107a, 107b) est formée entre chacun des éléments fonctionnels (110) et la partie pliée inférieure (106a), et entre la partie pliée inférieure (106a) et la partie pliée supérieure (106c) dans la région entre des parties fixes et dans lequel
le produit d'hygiène (1) est un article absorbant,
l'élément fonctionnel (110) est une partie de corps principal absorbante qui comporte une feuille supérieure, une feuille arrière et un corps absorbant qui est positionné entre la feuille supérieure et la feuille arrière, et
la paire de parties pliées (106b, 106b) sont une paire de parois anti-fuite.
